# EUROPÄISCHE PATENTSCHRIFT

(11) **EP 2 111 403 B1**
(45) Veröffentlichungstag und Bekanntmachung des Hinweises auf die Patenterteilung: **10.11.2010**
(21) Anmeldenummer: 07818574.1
(22) Anmeldetag: 29.09.2007
(51) Int. Cl.: C07D 487/04, A61K 31/55, A61P 35/00

(54) **TRIAZA-BENZO[E]AZULENDERIVATE ZUR BEHANDLUNG VON TUMOREN**
TRIAZABENZO[E]AZULENE DERIVATIVES FOR THE TREATMENT OF TUMORS
DÉRIVÉS DE TRIAZA-BENZO[E]AZULÈNE POUR LE TRAITEMENT DE TUMEURS

(30) Priorität: 03.11.2006 DE 102006051796
(43) Veröffentlichungstag der Anmeldung: 28.10.2009
(73) Patentinhaber: Merck Patent GmbH, 64293 Darmstadt (DE)
(72) Erfinder: HOELZEMANN, Guenter, 64342 Seeheim-Jugenheim (DE); GREINER, Hartmut, 64331 Weiterstadt (DE); AMENDT, Christiane, 64367 Muehltal/Trautheim (DE)
(86) Internationale Anmeldenummer: PCT/EP2007/008494
(87) Internationale Veröffentlichungsnummer: WO 2008/052628

(56) Entgegenhaltungen:
- WO-A-2007/079820
- DE-A1- 2 318 673
- KOSYCHOCA L ET AL: "SYNTHESIS OF SUBSTITUTED 5,6-DIHYDRO-4H-Ä1,2,4ÜTRIAZOLO-Ä4,3-aÜÄ1,5 ÜB ENZODIAZEPINES" CHEMISTRY OF HETEROCYCLIC COMPOUNDS (A TRANSLATION OF KHIMIYA GETEROTSIKLICHESKIKH SOEDINENII), PLENUM PRESS CO., NEW YORK, NY, US, Bd. 40, Nr. 6, 2004, Seiten 811-815, XP002433485 ISSN: 0009-3122 in der Anmeldung erwähnt

## Beschreibung

### HINTERGRUND DER ERFINDUNG

Der Erfindung lag die Aufgabe zugrunde, neue Verbindungen mit wertvollen Eigenschaften aufzufinden, insbesondere solche, die zur Herstellung von Arzneimitteln verwendet werden können.

Die vorliegende Erfindung betrifft Verbindungen und die Verwendung von Verbindungen, bei denen die Hemmung, Regulierung und/oder Modulation der Signaltransduktion von Kinasen, insbesondere der TGF-beta-Rezeptorkinasen, eine Rolle spielt, ferner pharmazeutische Zusammensetzungen, die diese Verbindungen enthalten, sowie die Verwendung der Verbindungen zur Behandlung kinasebedingter Krankheiten.

Transforming growth factor beta ist der Prototyp der TGF-beta Superfamilie, einer Familie von hoch konservierten, pleiotrophen Wachstumsfaktoren, die sowohl während der Embryonalentwicklung als auch im adulten Organismus wichtige Funktionen ausüben. In Säugetieren wurden drei Isoformen von TGF-beta (TGF-beta 1, 2 und 3) identifiziert, wobei TGF-beta 1, die häufigste Isoform darstellt (Kingsley (1994) Genes Dev 8:133-146). TGF-beta 3 wird z.B. nur in mesenchymalen Zellen exprimiert, wohingegen TGF-beta 1 in mesenchmalen und epithelialen Zellen gefunden wird. TGF-beta wird als Präproprotein synthetisiert und in inaktiver Form in die extrazelluläre Matrix abgegeben (Derynck (1985) Nature 316: 701-705; Bottinger (1996) PNAS 93: 5877-5882). Neben der abgespaltenen Proregion, die auch als Latency Associated Peptide (LAP) bezeichnet wird und mit der reifen Region assoziiert bleibt, kann auch eines der 4 Isoformen der Latent TGF-beta Binding Proteins (LTBP 1-4) an TGF-beta gebunden sein (Gentry (1988) Mol Cell Biol 8: 4162-4168, Munger (1997) Kindey Int 51: 1376-1382). Die für die Entfaltung der biologischen Wirkung von TGF-beta notwendige Aktivierung des inaktiven Komplexes ist noch nicht vollständig geklärt. Allerdings ist mit Sicherheit eine proteolytische Prozessierung z.B. durch Plasmin, Plasma Transglutaminase oder Thrombospondin notwendig (Munger (1997) Kindey Int 51: 1376-1382). Der aktivierte Ligand TGF-beta vermittelt seine biologische Wirkung über drei membranständige TGF-beta Rezeptoren, die ubiquitär exprimierten Typ I und Typ II Rezeptoren und den Typ III Rezeptoren Betaglycan und Endoglin, wobei letzterer nur in Endothelzellen exprimiert wird (Gougos (1990) J Biol Chem 264: 8361-8364, Loeps-Casillas (1994) J Cell Biol 124:557-568). Beide Typ III TGF-beta Rezeptoren besitzen keine intrazelluläre Kinasedomäne, die eine Signalweiterleitung in die Zelle ermöglicht. Da die Typ III TGF-beta Rezeptoren alle drei TGF-beta Isoformen mit hoher Affinität binden und auch Typ II TGF-beta Rezeptor eine höhere Affinität für an Typ III Rezeptor gebundenen Liganden besitzt, besteht die biologische Funktion vermutlich in der Regulation der Verfügbarkeit der Liganden für Typ I und Typ II TGF-beta Rezeptoren (Lastres (1996) J Cell Biol 133:1109-1121; Lopes-Casillas (1993) Cell 73: 1435-1344). Die strukturell eng verwandten Typ I und Typ II Rezeptoren besitzen im zytoplasmatischen Bereich eine Serin/Threonin-Kinasedomäne, die für die Signalweiterleitung verantwortlich ist. Typ II TGF-beta Rezeptor bindet TGF-beta, woraufhin der Typ I TGF-beta Rezeptor zu diesem signalweiterleitenden Komplex rekrutiert wird. Die Serin/Threonin Kinasedomäne des Typ II Rezeptors ist konstitutiv aktiv und kann in diesem Komplex Serylreste in der sogenannten GS-Domäne des Typ I Rezeptors phosphorylieren. Diese Phosphorylierung aktiviert die Kinase des Typ I Rezeptors, die nun ihrerseits intrazelluläre Signalmediatoren, die SMAD Proteine phosphorylieren kann und damit die intrazelluläre Signalweiterleitung initiiert (zusammengefasst in Derynck (1997) Biochim Biophys Acta 1333: F105-F150). Die Proteine der SMAD-Familie dienen als Substrate für alle TGF-beta Familien Rezeptor Kinasen. Bisher wurden 8 SMAD Proteine identifiziert, die sich in 3 Gruppen aufteilen: (1) Rezeptor-assozierte SMADs (R-SMADs) sind direkte Substrate der TGF-β Rezeptor Kinasen (SMAD1, 2, 3, 5, 8); (2) Co-SMADs, die mit den R-Smads während der Signalkaskade assoziieren (SMAD4); and (3) inhibitorische SMADs (SMAD6, 7), die die Aktivität der oben genannten SMAD Proteine hemmen. Von den verschiedenen R-SMADs, sind SMAD2 and SMAD3 die TGF-beta spezifischen Signalmediatoren. In der TGF-beta Signalkaskade werden also SMAD2/SMAD3 vom Typ I TGF-beta Rezeptor phosphoryliert, wodurch sie mit SMAD4 assoziieren können. Der entstandene Komplex aus SMAD2/SMAD3 und SMAD4 kann nun in den Zellkern translokalisert werden und dort direkt oder über andere Proteine die Transkription der TGF-beta regulierten Gene initiieren (zusammengefasst in Itoh (2000) Eur J Biochem 267: 6954-6967; Shi (2003) Cell 113: 685-700).

Das Spektrum der Funktionen von TGF-beta ist breitgefächert und abhängig von Zellart und Differenzierungsstatus (Roberts (1990) Handbook of Experimental Pharmacology: 419-472). Zu den zellulären Funktionen, die von TGF-beta beeinflusst werden, gehören: Apoptose, Proliferation, Differenzierung, Mobilität und Zelladhäsion. Dementsprechend spielt TGF-beta eine wichtige Rolle in den verschiedensten biologischen Prozessen. Während der Embryonalentwicklung wird es an Orten der Morphogenese und insbesondere an Stellen mit epithelialermesenchymaler Interaktion exprimiert und induziert dort wichtige Differenzierungsprozesse (Pelton (1991) J Cell Biol 115:1091-1105). Eine Schlüsselfunktion übt TGF-beta auch bei der Selbsterneuerung und Aufrechterhaltung eines undifferenzierten Zustandes von Stammzellen aus (Mishra (2005) Science 310: 68-71). Zudem erfüllt TGF-beta auch in der Regulation des Immunsystems wichtige Funktionen. Es wirkt im allgemeinen immunsuppressiv, da es u.a. die Proliferation von Lymphozyten hemmt und die Aktivität von Gewebsmakrophagen einschränkt. TGF-beta lässt so inflammatorische Reaktionen wieder abklingen und hilft so überschießende Immunreaktionen zu vermeiden (Bogdan (1993) Ann NY Acad Sci 685: 713-739, zusammengefasst in Letterio (1998) Annu Rev Immunol 16: 137-161). Eine andere Funktion von TGF-beta ist die Regulation der Zellproliferation. TGF-beta hemmt das Wachstum von Zellen endothelialer, epithelialer und hämatopoetischer Herkunft, fördert aber das Wachstum von Zellen mesenchymalen Ursprungs (Tucker (1984) Science 226:705-707, Shipley (1986) Cancer Res 46:2068-2071, Shipley (1985) PNAS 82: 4147-4151). Eine weitere wichtige Funktion von TGF-beta ist die Regulation zellulärer Adhäsion und von Zell-Zell-Interaktionen. TGF-beta fördert den Aufbau der extrazellulären Matrix durch die Induktion von Proteinen der extrazellulären Matrix, wie z.B. Fibronectin und Kollagen. Zusätzlich reduziert TGF-beta die Expression von matrixdegradierenden Metalloproteasen und Inhibitoren der Metalloproteasen (Roberts (1990) Ann NY Acad Sci 580: 225-232; Ignotz (1986) J Biol Chem 261: 4337-4345; Overall (1989) J Biol Chem 264: 1860-1869); Edwards (1987) EMBO J 6: 1899-1904).

Das breite Wirkungsspektrum von TGF-beta impliziert, dass TGF-beta eine wichtige Rolle bei vielen physiologischen Gegebenheiten, wie der Wundheilung und bei pathologischen Prozessen, wie Krebs und Fibrose, spielt.

TGF-beta ist eines der Schlüssel-Wachstumsfaktoren bei der Wundheilung (zusammengefasst in O'Kane (1997) Int J Biochem Cell Biol 29: 79-89). Während der Granulationsphase wird TGF-beta an der Verletzungsstelle aus Blutplättchen freigegeben. TGF-beta reguliert sodann seine eigene Produktion in Makrophagen und induziert die Sekretion von anderen Wachstumsfaktoren z.B. durch Monozyten. Die wichtigsten Funktionen während der Wundheilung beinhalten die Stimulierung der Chemotaxis von inflammatorischen Zellen, die

Synthese von extrazellulärer Matrix und die Regulation der Proliferation, Differenzierung und Genexpression aller wichtigen am Wundheilungsprozess beteiligten Zelltypen.

Unter pathologischen Bedingungen können diese TGF-beta vermittelte Effekte, insbesondere die Regulation der Produktion von extrazellulärer Matrix (ECM) zur Fibrose bzw. in der Haut zu Narben führen (Border (1994) N Engl J Med 331:1286-1292).

Für die fibrotischen Erkrankungen, diabetische Nephropathie und Glomeronephritis konnte nachgewiesen werden, dass TGF-beta renale Zellhypertrophie und die pathogene Akkumulation der extrazellulären Matrix fördert. Die Unterbrechung des TGF-beta Signalweges durch eine Behandlung mit anti-TGF-beta Antikörpern verhindert die Expansion der mesangialen Matrix, die progressive Abnahme der Nierenfunktion und reduziert etablierte Lesionen der diabetischen Glomerulopathie in diabetischen Tieren (Border (1990) 346: 371-374, Yu (2004) Kindney Int 66: 1774-1784, Fukasawah (2004) Kindney Int 65: 63-74, Sharma (1996) Diabetes 45: 522-530).

Auch bei der Leberfibrose spielt TGF-beta eine wichtige Rolle. Die für die Entwicklung der Leberfibrose wesentliche Aktivierung der hepatischen Sternzellen (engl. hepatic stellate cell) zu Myofibroblasten, den Hauptproduzent der extrazellulären Matrix im Rahmen der Entwicklung einer Leberzirrhose, wird von TGF-beta stimuliert. Auch hier konnte gezeigt werden, dass die Unterbrechung des TGF-beta Signalweges Fibrose in experimentellen Modellen reduziert (Yata (2002) Hepatology 35:1022-1030; Arias (2003) BMC Gastroenterol 3:29)

Eine Schlüsselfunktion nimmt TGF-beta auch bei der Krebsentstehung ein (zusammengefasst in Derynck (2001) Nature Genetics: 29: 117-129; Elliott (2005) J Clin Onc 23: 2078-2093). In frühen Stadien der Krebsentwicklung wirkt TGF-beta der Krebsentstehung entgegen. Diese tumorsupprimierende Wirkung beruht hauptsächlich auf der Fähigkeit von TGF-beta die Teilung von epithelialen Zellen zu inhibieren. Im Gegensatz dazu fördert TGF-beta Krebswachstum und Metastasenbildung in späten Tumorstadien. Dies lässt sich darauf zurückführen, dass die meisten epithelialen Tumoren eine Resistenz gegenüber der wachstumshemmenden Wirkung von TGF-beta entwickeln und TGF-beta gleichzeitig über andere Mechanismen das Wachstum der Krebszellen unterstützt. Zu diesen Mechanismen gehört die Förderung der Angiogenese, die immunsuppressive Wirkung, die Tumorzellen bei der Umgehung der Kontrollfunktion des Immunsystems (engl. immunosurveillance) unterstützt und die Förderung von Invasivität und Metastasenbildung. Die Ausbildung eines invasiven Phänotyps der Tumorzellen ist eine Hauptvoraussetzung für die Metastasenbildung. TGF-beta fördert diesen Prozess durch seine Fähigkeit die zelluläre Adhäsion, Motilität und die Ausbildung der extrazellulären Matrix zu regulieren. Weiterhin induziert TGF-beta die Umwandlung von einem epithelialen Phänotyp der Zelle zum invasiven mesenchymalen Phänotyp (engl. Epitheliale Mesenchymale Transition = EMT). Die wichtige Rolle, die TGF-beta bei der Förderung des Krebswachstums spielt, demonstrieren auch Untersuchungen, die eine Korrelation zwischen einer starken TGF-beta Expression und einer schlechten Prognose aufzeigen. Erhöhte TGF-beta Level wurden u.a. in Patienten mit Prostata-, Brust-, Darm- und Lungenkrebs gefunden (Wikström (1998) Prostate 37: 19-29; Hasegawa (2001) Cancer 91: 964-971; Friedman (1995), Cancer Epidemiol Biomarkers Prev. 4:549-54). Aufgrund der oben beschriebenen krebsfördernden Wirkungen von TGF-beta bietet sich die Hemmung des TGF-beta Signalweges, z.B. über die Hemmung des TGF-beta Typ I Rezeptors als therapeutisches Konzept an. In zahlreichen präklinischen Versuchen konnte gezeigt werden, dass in der Tat die Unterbrechung des TGF-beta Signalweges das Krebswachstum hemmt. So reduziert die Behandlung mit löslichem TGF-beta Typ II Rezeptor die Bildung von Metastasen in transgenen Mäusen, die im Laufe der Zeit invasiven Brustkrebs entwickeln (Muraoka (2002) J Clin Invest 109: 1551-1559, Yang (2002) J Clin Invest 109: 1607-1615). Tumorzellinien, die einen defekten TGF-beta Typ II Rezeptor exprimieren, zeigen verringertes Tumor- und Metastasenwachstum (Oft (1998) Curr Biol 8: 1243-1252, McEachern (2001) Int J Cancer 91:76-82, Yin (1999) Jclin Invest 103: 197-206).

Zustände, "die durch eine gesteigerte TGF-β-Aktivität gekennzeichnet sind", umfassen solche Zustände, wobei die TGF-β-Synthese so stimuliert ist, dass das TGF-β in erhöhten Spiegeln vorhanden ist, oder wobei das latente TGF-β-Protein unerwünscht aktiviert oder in das aktive TGF-β-Protein umgewandelt ist oder wobei die TGF-β-Rezeptoren hochreguliert sind oder wobei das TGF-β-Protein eine gesteigerte Bindung an Zellen oder an die extrazelluläre Matrix am Krankheitsherd aufweist. Somit betrifft in jedem Fall "gesteigerte Aktivität" einen beliebigen Zustand, bei dem die biologische Aktivität von TGF-β unabhängig von der Ursache unerwünscht hoch ist.

Eine Reihe von Erkrankungen wurden mit der Überproduktion von TGF-β1 in Zusammenhang gebracht. Inhibitoren des intrazellulären TGF-β-Signalwegs sind geeignete Behandlungen für fibroproliferative Erkrankungen. Fibroproliferative Erkrankungen umfassen spezifisch Nierenstörungen, die mit einer unregulierten TGF-β-Aktivität einhergehen, und starke Fibrose, einschließlich Glomerulonephritis (GN), wie mesangiale proliferative GN, Immun-GN und Halbmond-GN. Andere Nierenzustände umfassen diabetische Nephropathie, renale interstitielle Fibrose, renale Fibrose bei Transplantat-Patienten, die Cyclosporin erhalten, und mit HIV einhergehende Nephropathie. Collagen-Gefäßstörungen umfassen progressive systemische Sklerose, Polymyositis, Sklerodermie, Dermatomyositis, eosinophile Fascitis, Morphea oder solche Störungen, die mit dem Vorkommen des Raynaud-Syndroms einhergehen. Lungenfibrosen, die durch eine übermäßige TGF-β-Aktivität verursacht werden, umfassen das Atemstörungssyndrom bei Erwachsenen, idiopathische Lungenfibrose und interstitielle Lungenfibrose, die oft mit Autoimmunstörungen einhergeht, wie systemischer Lupus erythematodes und Sklerodermie, chemischer Kontakt oder Allergien. Eine weitere Autoimmunstörung, die mit fibroproliferativen Eigenschaften einhergeht, ist rheumatoide Arthritis.

Augenerkrankungen, die mit einem fibroproliferativen Zustand einhergehen, umfassen eine proliferative Vitreoretinopathie, die bei einer Wiederbefestigungsoperation der Retina vorkommt, Katarakt-Extraktion mit einer intraokularen Linsenimplantation, und Post-Glaukom-Drainagenoperation, und gehen mit einer TGF-β1-Überproduktion einher.

Fibrose-Erkrankungen, die mit einer TGF-β1-Überproduktion einhergehen, können in chronische Zustände, wie die Fibrose der Niere, Lunge und Leber, und akutere Zustände, wie Hautvernarbung und Restenose, unterteilt werden (Chamberlain, J. Cardiovascular Drug Reviews, 19(4): 329-344). Die Synthese und die Sekretion von TGF-β1 durch Tumorzellen können ebenfalls zur Immunsuppression führen, wie es bei Patienten mit aggressivem Gehirn oder Brusttumoren beobachtet wird (Arteaga, et al. (1993) J. Clin. Invest. 92: 2569-2576). Der Verlauf der Leishmania-Infektion bei Mäusen wird durch TGF-β1 drastisch verändert (Barral-Netto, et al. (1992) Science 257: 545-547). TGF-β1 verschlechterte die Krankheit, wohingegen TGF-β1-Antikörper den Fortschritt der Erkrankung in genetisch anfälligen Mäusen aufhielten. Genetisch resistente Mäuse wurden bei der Verabreichung von TGF-β1 gegenüber Leishmania-Infektion anfällig.

Die tiefreichenden Wirkungen auf die Ablagerung der extrazellulären Matrix wurden im Überblick dargestellt (Rocco und Ziyadeh (1991) in Contemporary Issues in Nephrology v.23, Hormones, autocoids and the kidney, Hrsg. Jay Stein, Churchill Livingston, New York S. 391-410; Roberts, et al. (1988) Rec. Prog. Hormone Res. 44: 157-197) und umfassen die Stimulation der Synthese und die Hemmung des Abbaus der extrazellulären Matrixkomponenten. Da die Struktur- und Filtrationseigenschaften des Glomerulus zum Großteil durch die extrazelluläre Matrix-Zusammensetzung des Mesangiums und der glomerulären Membran bestimmt werden, ist es nicht überraschend, dass TGF-β1 tiefreichende Wirkungen auf die Niere hat. Die Anreicherung der mesangialen Matrix bei der proliferativen Glomerulonephritis (Border, et al., (1990) Kidney Int. 37: 689-695) und der diabetischen Nephropathie (Mauer, et al. (1984) J. Clin. Invest. 74: 1143-1155) sind klare und dominante pathologische Merkmale der Erkrankungen. Die TGF-β1-Spiegel sind bei der diabetischen Glomerulosklerose beim Menschen (fortgeschrittene Neuropathie) erhöht (Yamamoto, et al. (1993) Proc. Natl. Acad. Sci. 90: 1814-1818). TGF-β1 ist ein wichtiger Vermittler bei der Genese der renalen Fibrose bei einer Reihe von Tiermodellen (Phan, et al. (1990) Kidney Int. 37: 426; Okuda, et al. (1990) J. Clin. Invest. 86: 453). Die Unterdrückung experimentell induzierter Glomerulonephritis in Ratten wurde durch Antiserum gegen TGF-β1 (Border, et al. (1990) Nature 346: 371) und durch ein extrazelluläres Matrixprotein, Decorin, das TGF-β1 binden kann, gezeigt (Border, et al. (1992) Nature 360: 361-363).

Zu viel TGF-β1 führt zur Bildung von Hautnarbengewebe. Das Neutralisieren von TGF-β1-Antikörpern, die in die Ränder heilender Wunden bei Ratten injiziert wurden, hemmte Befunden zufolge die Narbenbildung, ohne dass die Rate der Wundheilung oder die Zugfestigkeit der Wunde beeinträchtigt wurde (Shah, et al. (1992) Lancet 339: 213-214). Gleichzeitig war die Angiogenese niedriger, die Anzahl der Makrophagen und Monocyten in der Wunde geringer und das Ausmaß der disorganisierten Kollagenfaser-Ablagerung in dem Narbengewebe verringert.

TGF-β1 kann ein Faktor bei der progressiven Verdickung der Arterienwand sein, die von der Proliferation der glatten Muskelzellen und der Ablagerung von extrazellulärer Matrix in der Arterie nach der Ballongefäßplastik verursacht wird. Der Durchmesser der wiederverschlossenen Arterie kann durch diese Verdickung 90% reduziert sein, und da der Großteil der Reduktion des Durchmessers auf der extrazellulären Matrix und nicht auf den Körpern der glatten Muskelzellen beruht, kann man diese Gefäße wieder auf 50% öffnen, indem einfach die übermäßige Ablagerung der extrazellulären Matrix reduziert wird. Bei nicht verletzten Schweine-Arterien, die mit einem TGF-β1-Gen in vivo transfiziert wurden, ging die TGF-β1-Genexpression sowohl mit der Synthese der extrazellulärer Matrix als auch mit Hyperplasie einher (Nabel, et al. (1993) Proc. Natl. Acad. Sci USA 90: 10759-10763). Die durch TGF-β1 induzierte Hyperplasie war nicht so ausgiebig, wie diejenige, die mit PDGF-BB induziert wurde, jedoch war die extrazelluläre Matrix bei TGF-β1-Transfektanten ausgeprägter. Es gab keine Ablagerung extrazellulärer Matrix bei einer durch FGF-1 (einer sezernierten Form von FGF) induzierten Hyperplasie bei diesem Genübertragungsmodell beim Schwein (Nabel (1993) Nature 362: 844-846).

Es gibt verschiedene Arten von Krebs, wobei das vom Tumor erzeugte TGF-β1 schädlich sein kann. MATLyLu-Prostatakrebszellen bei der Ratte (Steiner und Barrack (1992) Mol. Endocrinol 6: 15-25) und MCF-7 Brustkrebszellen beim Menschen (Arteaga, et al. (1993) Cell Growth and Differ. 4: 193-201) wurden nach der Transfektion mit einem Vektor, der das Maus-TGF-β1 exprimierte, tumorigener und metastatischer. TGF-β1 ging mit Angiogenese, Metastase und schlechter Prognose bei Human-Prostata und fortgeschrittenem Darmkrebs einher (Wikstrom, P., et al. (1988) Prostate 37; 19-29; Saito, H., et al. (1999) Cancer 86: 1455-1462). Bei Brustkrebs geht eine schlechte Prognose mit erhöhtem TGF-β einher (Dickson, et al. (1987) Proc. Natl. Acad. Sci. USA 84: 837-841; Kasid, et al. (1987) Cancer Res. 47: 5733-5738; Daly, et al. (1990) J. Cell Biochem. 43: 199-211; Barrett-Lee, et al. (1990) Br. J. Cancer 61: 612-617; King, et al (1989) J. Steroid Biochem. 34: 133-138; Welch, et al (1990) Proc. Natl. Acad. Sci USA 87: 7678-7682; Walker et al. (1992) Eur. J. Cancer 238: 641-644), und die Induktion von TGF-β1 durch Tamoxifen-Behandlung (Butta, et al. (1992) Cancer Res. 52: 4261-4264) ging mit einem Versagen der Tamoxifen-Behandlung bei Brustkrebs einher (Thompson, et al. (1991) Br. J. Cancer 63: 609-614). Anti-TGF-β1-Antikörper hemmen das Wachstum von MDA-231-Human-Brustkrebszellen in athymischen Mäusen (Arteaga, et al. (1993) J. Clin. Invest. 92: 2569-2576), eine Behandlung, die mit einem Anstieg der natürlichen Killerzellaktivität in der Milz korreliert ist. CHO-Zellen, die mit latentem TGF-β1 transfiziert sind, zeigten ebenfalls gesenkte NK-Aktivität und gesteigertes Tumorwachstum in Nacktmäusen (Wallick, et al. (1990) J. Exp. Med. 172: 177-1784). Somit kann das durch Brusttumore sezernierte TGF-β eine endokrine Immunsuppression verursachen. Hohe Plasmakonzentrationen von TGF-β1 zeigen eine schlechte Prognose für Patienten mit fortgeschrittenem Brustkrebs (Anscher, et al. (1993) N. Engl. J. Med. 328: 1592-1598). Patienten mit hohem zirkulierendem TGF-β vor der Hochdosis-Chemotherapie und autologer Knochenmarktransplantation haben ein hohes Risiko für eine hepatische venookklusive Erkrankung (15-50% sämtlicher Patienten mit einer Mortalitätsrate bis zu 50%) und eine idiopathische interstitielle Pneumonitis (40 bis 60% sämtlicher Patienten). Die Bedeutung dieser Befunde ist, dass 1) erhöhte Plasmaspiegel von TGF-β1 zur Identifikation von Risikopatienten verwendet werden können, und dass 2) eine Reduktion von TGF-β1 die Morbidität und Mortalität dieser üblichen Behandlungen für Brustkrebspatienten senken kann.

Viele maligne Zellen sezernieren transformierenden Wachstumsfaktor β (TGF-β), ein leistungsfähiges Immunsuppressivum, was nahe legt, dass die TGF-β-Produktion einen signifikanten Tumor-Escape-Mechanismus vor der Wirts-Immunüberwachung darstellen kann. Die Errichtung einer Leukocyten-Subpopulation mit unterbrochenem TGF-β-Signalweg in dem tumortragenden Wirt bietet eine leistungsfähige Maßnahme zur Immuntherapie von Krebs. Ein transgenes Tiermodell mit unterbrochenem TGF-β-Signalweg in T-Zellen kann einen normal letalen TGF-β-überexprimierenden Lymphom-Tumor, EL4, auslöschen (Gorelik und Flavell, (2001) Nature Medicine 7(10): 1118-1122). Das Herunterregulieren der TGF-β-Sekretion in Tumorzellen führt zur Wiederherstellung der Immunogenität im Wirt, wohingegen die T-Zell-Unempfindlichkeit gegenüber TGF-β zu einer beschleunigten Differenzierung und Autoimmunität führt, deren Elemente erforderlich sein können, um die Self-Antigen-exprimierenden Tumore in einem tolerant gemachten Wirt zu bekämpfen. Die immunsuppressiven Wirkungen von TGF-β sind auch bei einer Subpopulation von HIV-Patienten mit einer niedrigeren lmmunreaktion als vorhergesagt auf der Basis ihrer CD4/CD8-T-Zellzahlen beteiligt (Garba, et al., J. Immunology (2002) 168: 2247-2254). Ein TGF-β-neutralisierender Antikörper konnte die Wirkung in der Kultur umkehren, was anzeigt, dass sich TGF-β-Signalweg-Inhibitoren bei der Umkehr der Immunsuppression, die bei diesem Anteil von HIV-Patienten vorliegt, eignen können.

Während der frühesten Stufen der Karzinogenese kann TGF-β1 als leistungsfähiger Tumorsuppressor wirken und kann die Wirkungen einiger chemopräventiver Mittel vermitteln. Bei einem gewissen Punkt während der Entwicklung und des Verlaufs maligner Neoplasmen scheinen sich Tumorzellen von der TGF-β-abhängigen Wachstumshemmung parallel zum Erscheinen von biologisch aktivem TGF-β in der Mikroumgebung zu entziehen. Die doppelte Tumorsuppressions- bzw. Tumorförderungs-Rolle von TGF-β wurde am deutlichsten in einem transgenen System gezeigt, das TGF-β in Keratinocyten überexprimiert. Transgene waren zwar gegenüber der Bildung gutartiger Hautläsionen resistenter, jedoch war die Rate der Metastasen-Konversion bei den Transgenen drastisch erhöht (Cui, et al. (1996) Cell 86(4): 531-42). Die Produktion von TGF-β1 durch maligne Zellen in primären Tumoren scheint mit fortschreitenden Stufen der Tumorprogression zu steigen. Untersuchungen bei vielen Hauptepithelkrebsarten legen nahe, dass die erhöhte Produktion von TGF-β durch Krebs beim Mensch als relativ spätes Ereignis während der Tumorprogression eintritt. Zudem verhilft dieses tumorassoziierte TGF-β den Tumorzellen zu einem selektiven Vorteil und fördert die Tumorprogression. Die Wirkungen von TGF-β auf Zell-Zell- und Zell-Stroma-Wechselwirkungen führt zu einer größeren Neigung für die Invasion und Metastase. Tumorassoziiertes TGF-β kann es Tumorzellen ermöglichen, sich der Immun überwachung zu entziehen, da es ein leistungsfähiger Inhibitor der klonalen Expansion aktivierter Lymphocyten ist. Es wurde auch gezeigt, dass TGF-β die Produktion von Angiostatin hemmt. Krebstherapie-Modalitäten, wie Strahlungstherapie und Chemotherapie, induzieren die Produktion von aktiviertem TGF-β im Tumor, wodurch das Auswachsen maligner Zellen selektiert wird, die gegenüber TGF-βwachstumsinhibitorischen Wirkungen resistent sind. Somit steigern diese Antikrebs-Behandlungen die Gefahr und beschleunigen die Entwicklung von Tumoren mit gesteigertem Wachstum und Invasionsvermögen. In dieser Situation können Mittel, die die TGF-β-vermittelte Signaltransduktion ansteuern, eine sehr effiziente Therapiestrategie sein. Es wurde gezeigt, dass die Resistenz der Tumorzellen gegenüber TGF-β einen Großteil der cytotoxischen Wirkungen der Strahlungstherapie und Chemotherapie unwirksam macht, und die behandlungsabhängige Aktivierung von TGF-β im Stroma kann sogar schädlich sein, da es die Mikroumgebung gegenüber der Tumorprogression leitfähiger macht und zur Gewebeschädigung beiträgt, was zu Fibrose führt. Die Entwicklung von TGF-β-Signaftransduktionsinhibitoren hat wahrscheinlich einen Vorteil für die Behandlung von fortgeschrittenem Krebs allein und in Kombination mit anderen Therapien.

Die Verbindungen eignen sich zur Behandlung von Krebs und anderen Erkrankungszuständen, die durch TGF-β beeinflusst werden, durch Hemmen von TGF-β in einem Patienten, der dieses benötigt, indem dem Patient die Verbindung(en) verabreicht wird bzw. werden. TGF-β ist auch geeignet gegen Atherosklerose- (T.A. McCaffrey: TGF-βs and TGF-β Receptors in Atherosclerosis: Cytokine and Growth Factor Reviews 2000, 11, 103-114) und Alzheimer-Erkrankungen (Masliah, E.; Ho, G.; Wyss-Coray, T.: Functional Role of TGF-β in Alzheimer's Disease Microvascular Injury: Lessons from Transgenic Mice: Neurochemistry International 2001, 39, 393-400).

Es wurde gefunden, daß die erfindungsgemäßen Verbindungen und ihre Salze bei guter Verträglichkeit sehr wertvolle pharmakologische Eigenschaften besitzen.
Insbesondere zeigen sie TGFβ-Rezeptor I-Kinase inhibierende Eigenschaften.

Die erfindungsgemäßen Verbindungen zeigen bevorzugt eine vorteilhafte biologische Aktivität, die in auf Enzymen basierenden Assays, zum Beispiel Assays wie hierin beschrieben, leicht nachweisbar ist. In derartigen auf Enzymen basierenden Assays zeigen und bewirken die erfindungsgemäßen Verbindungen bevorzugt einen inhibierenden Effekt, der gewöhnlich durch IC₅₀-Werte in einem geeigneten Bereich, bevorzugt im mikromolaren Bereich und bevorzugter im nanomolaren Bereich dokumentiert wird.

Wie hierin besprochen, sind diese Signalwege für verschiedene Erkrankungen relevant. Dementsprechend sind die erfindungsgemäßen Verbindungen nützlich bei der Prophylaxe und/oder Behandlung von Erkrankungen, die von den genannten Signalwegen durch Interaktion mit einem oder mehreren der genannten Signalwege abhängig sind. Gegenstand der vorliegenden Erfindung sind deshalb erfindungsgemäße Verbindungen als Promotoren oder Inhibitoren, bevorzugt als Inhibitoren der hierin beschriebenen Signalwege. Bevorzugter Gegenstand der Erfindung sind deshalb erfindungsgemäße Verbindungen als Promotoren oder Inhibitoren, bevorzugt als Inhibitoren des TGFβ-Signalwegs.

Ein weiterer Gegenstand der vorliegenden Erfindung ist die Verwendung einer oder mehrerer erfindungsgemäßer Verbindungen bei der Behandlung und/oder Prophylaxe von Erkrankungen, bevorzugt den hier beschriebenen Erkrankungen, die durch eine gesteigerte TGFβ-Aktivität verursacht, vermittelt und/oder propagiert werden.

Gegenstand der vorliegenden Erfindung sind deshalb erfindungsgemäße Verbindungen als Arzneimittel und/oder Arzneimittelwirkstoffe bei der Behandlung und/oder Prophylaxe der genannten Erkrankungen und die Verwendung von erfindungsgemäßen Verbindungen zur Herstellung eines Pharmazeutikums für die Behandlung und/oder Prophylaxe der genannten Erkrankungen wie auch ein Verfahren zur Behandlung der genannten Erkrankungen umfassend die Verabreichung eines oder mehrerer erfindungsgemäßer Verbindungen an einen Patienten mit Bedarf an einer derartigen Verabreichung.

Der Wirt oder Patient kann jeglicher Säugerspezies angehören, z. B. einer Primatenspezies, besonders Menschen; Nagetieren, einschließlich Mäusen, Ratten und Hamstern; Kaninchen; Pferden, Rindern, Hunden, Katzen usw. Tiermodelle sind für experimentelle Untersuchungen von Interesse, wobei sie ein Modell zur Behandlung einer Krankheit des Menschen zur Verfügung stellen.

Die Suszeptibilität einer bestimmten Zelle gegenüber der Behandlung mit den erfindungsgemäßen Verbindungen kann durch Testen in vitro bestimmt werden. Typischerweise wird eine Kultur der Zelle mit einer erfindungsgemäßen Verbindung bei verschiedenen Konzentrationen für eine Zeitdauer kombiniert, die ausreicht, um den aktiven Mitteln zu ermöglichen, Zelltod zu induzieren oder Migration zu inhibieren, gewöhnlich zwischen ungefähr einer Stunde und einer Woche. Zum Testen in vitro können kultivierte Zellen aus einer Biopsieprobe verwendet werden. Die nach der Behandlung zurückbleibenden lebensfähigen Zellen werden dann gezählt.
Die Dosis variiert abhängig von der verwendeten spezifischen Verbindung, der spezifischen Erkrankung, dem Patientenstatus usw.. Typischerweise ist eine therapeutische Dosis ausreichend, um die unerwünschte Zellpopulation im Zielgewebe erheblich zu vermindern, während die Lebensfähigkeit des Patienten aufrechterhalten wird. Die Behandlung wird im Allgemeinen fortgesetzt, bis eine erhebliche Reduktion vorliegt, z. B. mindestens ca. 50 % Verminderung der Zelllast und kann fortgesetzt werden, bis im Wesentlichen keine unerwünschten Zellen mehr im Körper nachgewiesen werden.

Zur Identifizierung eines Signalübertragungswegs und zum Nachweis von Wechselwirkungen zwischen verschiedenen Signalübertragungswegen wurden von verschiedenen Wissenschaftlern geeignete Modelle oder Modellsysteme entwickelt, z.B. Zellkulturmodelle (z.B. Khwaja et al., EMBO, 1997, 16, 2783-93) und Modelle transgener Tiere (z.B. White et al., Oncogene, 2001, 20, 7064-7072). Zur Bestimmung bestimmter Stufen in der Signalübertragungskaskade können wechselwirkende Verbindungen genutzt werden, um das Signal zu modulieren (z.B. Stephens et al., Biochemical J., 2000, 351, 95-105). Die erfindungsgemäßen Verbindungen können auch als Reagenzien zur Testung kinaseabhängiger Signalübertragungswege in Tieren und/oder Zellkulturmodellen oder in den in dieser Anmeldung genannten klinischen Erkrankungen verwendet werden.

Die Messung der Kinaseaktivität ist eine dem Fachmann wohlbekannte Technik. Generische Testsysteme zur Bestimmung der Kinaseaktivität mit Substraten, z.B. Histon (z.B. Alessi et al., FEBS Lett. 1996, 399, 3, Seiten 333-338) oder dem basischen Myelinprotein sind in der Literatur beschrieben (z.B. Campos-González, R. und Glenney, Jr., J.R. 1992, J. Biol. Chem. 267, Seite 14535).

Zur Identifikation von Kinase-Inhibitoren stehen verschiedene Assay-Systeme zur Verfügung. Beim Scintillation-Proximity-Assay (Sorg et al., J. of. Biomolecular Screening, 2002, 7, 11-19) und dem FlashPlate-Assay wird die radioaktive Phosphorylierung eines Proteins oder Peptids als Substrat mit γATP gemessen. Bei Vorliegen einer inhibitorischen Verbindung ist kein oder ein vermindertes radioaktives Signal nachweisbar. Ferner sind die Homogeneous Time-resolved Fluorescence Resonance Energy Transfer- (HTR-FRET-) und Fluoreszenzpolarisations- (FP-) Technologien als Assay-Verfahren nützlich (Sills et al., J. of Biomolecular Screening, 2002, 191-214).

Andere nicht radioaktive ELISA-Assay-Verfahren verwenden spezifische Phospho-Antikörper (Phospho-AK). Der Phospho-AK bindet nur das phosphorylierte Substrat. Diese Bindung ist mit einem zweiten Peroxidasekonjugierten Anti-Schaf-Antikörper durch Chemilumineszenz nachweisbar (Ross et al., 2002, Biochem. J., unmittelbar vor der Veröffentlichung, Manuskript BJ20020786).

### STAND DER TECHNIK

Triazolo-1,5-benzodiazepine kennt man aus DE 2 318 673.
L. Kosychova et al. beschreiben in Chemistry of Heterocyclic Compounds, Vol. 40, 811-815 (2004) andere 5,6-Dihydro-4*H*-[1,2,4]triazolo-*a*][1,5]-benzodiazepine zur Tumorbekämpfung.
V. Ambrogi et al. beschreiben in J. Heterocyclic Chem. 31, 1349-1352 (1994) schwefelhaltige 4,5-Dihydro-*s*-triazolo[3,4-*d*][1,5]benzothiazepin-derivate.
V. Ambrogi et al. beschreiben in II Farmaco 48, 665-676 (1993) 1,4-Benzothiazine und 1,5-Benzothiazepine mit Wirkung auf das Zentralnervensystem.
Andere Triazolderivate sind als TGF-beta-Inhibitoren in WO 03/042211 A1 offenbart.
Wiederum andere Triazolderivate kennt man als TGF-beta-Inhibitoren aus WO 2004/026307 A1.
Biyclische Pyrrolderivate sind als TGF-beta-Inhibitoren in WO 02/094833 beschrieben.

### ZUSAMMENFASSUNG DER ERFINDUNG

Die Erfindung betrifft Verbindungen der Formel I 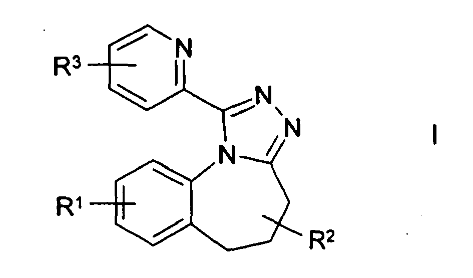 worin
- R¹: H, A, OH, OA, NO₂, NH₂, NHA, NA₂, Hal, CN, A-COO, COOH, COOA oder CONR⁴R⁵,
- R², R³: jeweils unabhängig voneinander H, A, Alkenyl mit 2-6 C- Atomen, Alkinyl mit 2-6 C-Atomen oder Hal,
- R⁴, R⁵: jeweils unabhängig voneinander H oder A,
- A: unverzweigtes oder verzweigtes Alkyl mit 1, 2, 3, 4, 5, 6, 7, 8, 9 oder 10 C-Atomen, worin 1-7 H-Atome durch F ersetzt sein können,
- Hal: F, Cl, Br oder I,
bedeuten,
sowie ihre pharmazeutisch verwendbaren Derivate, Solvate, Salze, Tautomere und Stereoisomere, einschließlich deren Mischungen in allen Verhältnissen.

Gegenstand der Erfindung sind auch die optisch aktiven Formen (Stereoisomeren), die Enantiomeren, die Racemate, die Diastereomeren sowie die Hydrate und Solvate dieser Verbindungen. Unter Solvate der Verbindungen werden Anlagerungen von inerten Lösungsmittelmolekülen an die Verbindungen verstanden, die sich aufgrund ihrer gegenseitigen Anziehungskraft ausbilden. Solvate sind z.B. Mono- oder Dihydrate oder Alkoholate.

Der Ausdruck "wirksame Menge" bedeutet die Menge eines Arzneimittels oder eines pharmazeutischen Wirkstoffes, die eine biologische oder medizinische Antwort in einem Gewebe, System, Tier oder Menschen hervorruft, die z.B. von einem Forscher oder Mediziner gesucht oder erstrebt wird.

Darüberhinaus bedeutet der Ausdruck "therapeutisch wirksame Menge" eine Menge, die, verglichen zu einem entsprechenden Subjekt, das diese Menge nicht erhalten hat, folgendes zur Folge hat:
verbesserte Heilbehandlung, Heilung, Prävention oder Beseitigung einer Krankheit, eines Krankheitsbildes, eines Krankheitszustandes, eines Leidens, einer Störung oder von Nebenwirkungen oder auch die Verminderung des Fortschreitens einer Krankheit, eines Leidens oder einer Störung.
Die Bezeichnung "therapeutisch wirksame Menge" umfaßt auch die Mengen, die wirkungsvoll sind, die normale physiologische Funktion zu erhöhen.

Gegenstand der Erfindung ist auch die Verwendung von Mischungen der erfindungsgemäßen Verbindungen, z.B. Gemische zweier Diastereomerer z.B. im Verhältnis 1:1, 1:2, 1:3, 1:4, 1:5, 1:10, 1:100 oder 1:1000. Besonders bevorzugt handelt es sich dabei um Mischungen stereoisomerer Verbindungen.

Gegenstand der Erfindung sind die Verbindungen der Formel I und ihre Salze sowie ein Verfahren zur Herstellung von Verbindungen der Formel I sowie ihrer pharmazeutisch verwendbaren Derivate, Salze, Solvate, Tautomeren und Stereoisomeren, **dadurch gekennzeichnet, daß** man
a) eine Verbindung der Formel II 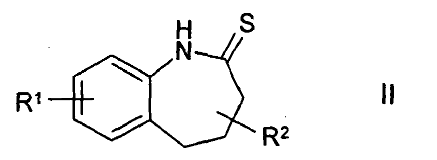 worin R¹ und R² die in Anspruch 1 angegebene Bedeutung hat,
   mit einer Verbindung der Formel III 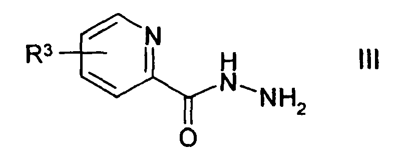 worin R³ die in Anspruch 1 angegebene Bedeutung hat,
   umsetzt,
   oder
b) einen Rest R¹ in einen anderen Rest R¹ umwandelt, indem man einen Ether spaltet,
   und/oder
   eine Base oder Säure der Formel I in eines ihrer Salze umwandelt.

Vor- und nachstehend haben die Reste R¹, R² und R³ die bei der Formel I angegebenen Bedeutungen, sofern nicht ausdrücklich etwas anderes angegeben ist.

A bedeutet Alkyl, ist unverzweigt (linear) oder verzweigt, und hat 1, 2, 3, 4, 5, 6, 7, 8, 9 oder 10 C-Atome. A bedeutet vorzugsweise Methyl, weiterhin Ethyl, Propyl, Isopropyl, Butyl, Isobutyl, sek.-Butyl oder tert.-Butyl, ferner auch Pentyl, 1-, 2- oder 3-Methylbutyl, 1,1- , 1,2- oder 2,2-Dimethylpropyl, 1-Ethylpropyl, Hexyl, 1- , 2- , 3- oder 4-Methylpentyl, 1,1- , 1,2- , 1,3- , 2,2- , 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl, weiter bevorzugt z.B. Trifluormethyl.
A bedeutet ganz besonders bevorzugt Alkyl mit 1, 2, 3, 4, 5 oder 6 C-Atomen, vorzugsweise Methyl, Ethyl, Propyl, Isopropyl, Butyl, Isobutyl, sek.-Butyl, tert.-Butyl, Pentyl, Hexyl, Trifluormethyl, Pentafluorethyl oder 1,1,1-Trifluorethyl.

R¹ bedeutet vorzugsweise H, OH, OA, Hal, CN oder A-COO.
R² bedeutet vorzugsweise H oder A.
R³ bedeutet vorzugsweise A.

Für die gesamte Erfindung gilt, daß sämtliche Reste, die mehrfach auftreten, gleich oder verschieden sein können, d.h. unabhängig voneinander sind.
Die Verbindungen der Formel I können ein oder mehrere chirale Zentren besitzen und daher in verschiedenen stereoisomeren Formen vorkommen. Die Formel I umschließt alle diese Formen.

Dementsprechend sind Gegenstand der Erfindung insbesondere diejenigen Verbindungen der Formel I, in denen mindestens einer der genannten Reste eine der vorstehend angegebenen bevorzugten Bedeutungen hat. Einige bevorzugte Gruppen von Verbindungen können durch die folgenden Teilformeln Ia bis Id ausgedrückt werden, die der Formel I entsprechen und worin die nicht näher bezeichneten Reste die bei der Formel I angegebene Bedeutung haben, worin jedoch

| | | |
|---|---|---|
| in Ia | R¹ | H, OH, OA, Hal, CN oder A-COO |
| | bedeutet; | |
| | | |
| in Ib | R² | H oder A bedeutet; |
| | | |
| in Ic | R³ | A bedeutet; |
| | | |
| in Id | R¹ | H, OH, OA, Hal, CN oder A-COO, |
| | R² | H oder A, |
| | R³ | A, |
| | A | unverzweigtes oder verzweigtes Alkyl mit 1, 2, 3, 4, 5 oder 6 C-Atomen, worin 1-5 H-Atome durch F ersetzt sein können, |
| | Hal | F, Cl, Br oder I, |

bedeuten;
sowie ihre pharmazeutisch verwendbaren Derivate, Salze, Solvate, Tautomere und Stereoisomere, einschließlich deren Mischungen in allen Verhältnissen.

Die Verbindungen der Formel I und auch die Ausgangsstoffe zu ihrer Herstellung werden im übrigen nach an sich bekannten Methoden hergestellt, wie sie in der Literatur (z.B. in den Standardwerken wie Houben-Weyl, Methoden der organischen Chemie, Georg-Thieme-Verlag, Stuttgart) beschrieben sind, und zwar unter Reaktionsbedingungen, die für die genannten Umsetzungen bekannt und geeignet sind. Dabei kann man auch von an sich bekannten, hier nicht näher erwähnten Varianten Gebrauch machen.

Die Ausgangsverbindungen der Formeln II und III sind in der Regel bekannt. Sind sie neu, so können sie aber nach an sich bekannten Methoden hergestellt werden.

Verbindungen der Formel I können vorzugsweise erhalten werden, indem man eine Verbindung der Formel II mit einer Verbindung der Formel III umsetzt.
Die Umsetzung erfolgt in einem inerten Lösungsmittel. Die Reaktionszeit liegt je nach den angewendeten Bedingungen zwischen einigen Minuten und 14 Tagen, die Reaktionstemperatur zwischen etwa 0° und 160°, normalerweise zwischen 20° und 150°, insbesondere zwischen etwa 80° und etwa 130°.
Als inerte Lösungsmittel eignen sich z.B. Kohlenwasserstoffe wie Hexan, Petrolether, Benzol, Toluol oder Xylol; chlorierte Kohlenwasserstoffe wie Trichlorethylen, 1,2-Dichlorethan,Tetrachlorkohlenstoff, Chloroform oder Dichlormethan; Alkohole wie Methanol, Ethanol, Isopropanol, n-Propanol, n-Butanol oder tert.-Butanol; Ether wie Diethylether, Diisopropylether, Tetrahydrofuran (THF) oder Dioxan; Glykolether wie Ethylenglykolmonomethyl- oder -monoethylether (Methylglykol oder Ethylglykol), Ethylenglykoldimethylether (Diglyme); Ketone wie Aceton oder Butanon; Amide wie Acetamid, Dimethylacetamid, 1-Methyl-pyrrolidinon (NMP) oder Dimethylformamid (DMF); Nitrile wie Acetonitril; Sulfoxide wie Dimethylsulfoxid (DMSO); Schwefelkohlenstoff; Carbonsäuren wie Ameisensäure oder Essigsäure; Nitroverbindungen wie Nitromethan oder Nitrobenzol; Ester wie Ethylacetat oder Gemische der genannten Lösungsmittel. Besonders bevorzugt ist n-Butanol, NMP und/oder DMF.

Zur Spaltung von Ethern eignet sich die Behandlung mit Bortribromid unter Standardbedingungen.

### Pharmazeutische Salze und andere Formen

Die genannten erfindungsgemäßen Verbindungen lassen sich in ihrer endgültigen Nichtsalzform verwenden. Andererseits umfaßt die vorliegende Erfindung auch die Verwendung dieser Verbindungen in Form ihrer pharmazeutisch unbedenklichen Salze, die von verschiedenen organischen und anorganischen Säuren und Basen nach fachbekannten Vorgehensweisen abgeleitet werden können. Pharmazeutisch unbedenkliche Salzformen der erfindungsgemäßen Verbindungen werden größtenteils konventionell hergestellt. Sofern die erfindungsgemäße Verbindung eine Carbonsäuregruppe enthält, läßt sich eines ihrer geeigneten Salze dadurch bilden, daß man die Verbindung mit einer geeigneten Base zum entsprechenden Basenadditionssalz umsetzt. Solche Basen sind zum Beispiel Alkalimetallhydroxide, darunter Kaliumhydroxid, Natriumhydroxid und Lithiumhydroxid; Erdalkalimetallhydroxide wie Bariumhydroxid und Calciumhydroxid; Alkalimetallalkoholate, z.B. Kaliumethanolat und Natriumpropanolat; sowie verschiedene organische Basen wie Piperidin, Diethanolamin und N-Methylglutamin. Die Aluminiumsalze der erfindungsgemäßen Verbindungen zählen ebenfalls dazu. Bei bestimmten erfindungsgemäßen Verbindungen lassen sich Säureadditionssalze dadurch bilden, daß man diese Verbindungen mit pharmazeutisch unbedenklichen organischen und anorganischen Säuren, z.B. Halogenwasserstoffen wie Chlorwasserstoff, Bromwasserstoff oder Jodwasserstoff, anderen Mineralsäuren und ihren entsprechenden Salzen wie Sulfat, Nitrat oder Phosphat und dergleichen sowie Alkyl- und Monoarylsulfonaten wie Ethansulfonat, Toluolsulfonat und Benzolsulfonat, sowie anderen organischen Säuren und ihren entsprechenden Salzen wie Acetat, Trifluoracetat, Tartrat, Maleat, Succinat, Citrat, Benzoat, Salicylat, Ascorbat und dergleichen behandelt. Dementsprechend zählen zu pharmazeutisch unbedenklichen Säureadditionssalzen der erfindungsgemäßen Verbindungen die folgenden: Acetat, Adipat, Alginat, Arginat, Aspartat, Benzoat, Benzolsulfonat (Besylat), Bisulfat, Bisulfit, Bromid, Butyrat, Kampferat, Kampfersulfonat, Caprylat, Chlorid, Chlorbenzoat, Citrat, Cyclopentanpropionat, Digluconat, Dihydrogenphosphat, Dinitrobenzoat, Dodecylsulfat, Ethansulfonat, Fumarat, Galacterat (aus Schleimsäure), Galacturonat, Glucoheptanoat, Gluconat, Glutamat, Glycerophosphat, Hemisuccinat, Hemisulfat, Heptanoat, Hexanoat, Hippurat, Hydrochlorid, Hydrobromid, Hydroiodid, 2-Hydroxyethansulfonat, Iodid, Isethionat, Isobutyrat, Lactat, Lactobionat, Malat, Maleat, Malonat, Mandelat, Metaphosphat, Methansulfonat, Methylbenzoat, Monohydrogenphosphat, 2-Naphthalinsulfonat, Nicotinat, Nitrat, Oxalat, Oleat, Pamoat, Pectinat, Persulfat, Phenylacetat, 3-Phenylpropionat, Phosphat, Phosphonat, Phthalat, was jedoch keine Einschränkung darstellt.

Weiterhin zählen zu den Basensalzen der erfindungsgemäßen Verbindungen Aluminium-, Ammonium-, Calcium-, Kupfer-, Eisen(III)-, Eisen(II)-, Lithium-, Magnesium-, Mangan(III)-, Mangan(II), Kalium-, Natrium- und Zinksalze, was jedoch keine Einschränkung darstellen soll. Bevorzugt unter den oben genannten Salzen sind Ammonium; die Alkalimetallsalze Natrium und Kalium,sowie die Erdalkalimetalsalze Calcium und Magnesium. Zu Salzen der erfindungsgemäßen Verbindungen, die sich von pharmazeutisch unbedenklichen organischen nicht-toxischen Basen ableiten, zählen Salze primärer, sekundärer und tertiärer Amine, substituierter Amine, darunter auch natürlich vorkommender substituierter Amine, cyclischer Amine sowie basischer Ionenaustauscherharze, z.B. Arginin, Betain, Koffein, Chlorprocain, Cholin, N,N'-Dibenzylethylendiamin (Benzathin), Dicyclohexylamin, Diethanolamin, Diethylamin, 2-Diethylaminoethanol, 2-Dimethylaminoethanol, Ethanolamin, Ethylendiamin, N-Ethylmorpholin, N-Ethylpiperidin, Glucamin, Glucosamin, Histidin, Hydrabamin, Iso-propylamin, Lidocain, Lysin, Meglumin, N-Methyl-D-glucamin, Morpholin, Piperazin, Piperidin, Polyaminharze, Procain, Purine, Theobromin, Triethanolamin, Triethylamin, Trimethylamin, Tripropylamin sowie Tris-(hydroxymethyl)-methylamin (Tromethamin), was jedoch keine Einschränkung darstellen soll.

Verbindungen der vorliegenden Erfindung, die basische stickstoffhaltige Gruppen enthalten, lassen sich mit Mitteln wie (C₁-C₄) Alkylhalogeniden, z.B. Methyl-, Ethyl-, Isopropyl- und tert.-Butylchlorid, -bromid und -iodid; Di(C₁-C₄)Alkylsulfaten, z.B. Dimethyl-, Diethyl- und Diamylsulfat; (C₁₀-C₁₈)Alkylhalogeniden, z.B. Decyl-, Dodecyl-, Lauryl-, Myristyl- und Stearylchlorid, -bromid und -iodid; sowie Aryl-(C₁-C₄)Alkylhalogeniden, z.B. Benzylchlorid und Phenethylbromid, quarternisieren. Mit solchen Salzen können sowohl wasser- als auch öllösliche erfindungsgemäße Verbindungen hergestellt werden.

Zu den oben genannten pharmazeutischen Salzen, die bevorzugt sind, zählen Acetat, Trifluoracetat, Besylat, Citrat, Fumarat, Gluconat, Hemisuccinat, Hippurat, Hydrochlorid, Hydrobromid, Isethionat, Mandelat, Meglumin, Nitrat, Oleat, Phosphonat, Pivalat, Natriumphosphat, Stearat, Sulfat, Sulfosalicylat, Tartrat, Thiomalat, Tosylat und Tromethamin, was jedoch keine Einschränkung darstellen soll.

Die Säureadditionssalze basischer Verbindungen der erfindungsgemäßen Verbindungen werden dadurch hergestellt, daß man die freie Basenform mit einer ausreichenden Menge der gewünschten Säure in Kontakt bringt, wodurch man auf übliche Weise das Salz darstellt. Die freie Base läßt sich durch In-Kontakt-Bringen der Salzform mit einer Base und Isolieren der freien Base auf übliche Weise regenerieren. Die freien Basenformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Basenformen. Wie erwähnt werden die pharmazeutisch unbedenklichen Basenadditionssalze der erfindungsgemäßen Verbindungen mit Metallen oder Aminen wie Alkalimetallen und Erdalkalimetallen oder organischen Aminen gebildet. Bevorzugte Metalle sind Natrium, Kalium, Magnesium und Calcium. Bevorzugte organische Amine sind N,N'-Dibenzylethylendiamin, Chlorprocain, Cholin, Diethanolamin, Ethylendiamin, N-Methyl-D-glucamin und Procain.

Die Basenadditionssalze von erfindungsgemäßen sauren Verbindungen werden dadurch hergestellt, daß man die freie Säureform mit einer ausreichenden Menge der gewünschten Base in Kontakt bringt, wodurch man das Salz auf übliche Weise darstellt. Die freie Säure läßt sich durch In-Kontakt-Bringen der Salzform mit einer Säure und Isolieren der freien Säure auf übliche Weise regenerieren. Die freien Säureformen unterscheiden sich in gewissem Sinn von ihren entsprechenden Salzformen in bezug auf bestimmte physikalische Eigenschaften wie Löslichkeit in polaren Lösungsmitteln; im Rahmen der Erfindung entsprechen die Salze jedoch sonst ihren jeweiligen freien Säureformen.

Enthält eine erfindungsgemäße Verbindung mehr als eine Gruppe, die solche pharmazeutisch unbedenklichen Salze bilden kann, so umfaßt die Erfindung auch mehrfache Salze. Zu typischen mehrfachen Salzformen zählen zum Beispiel Bitartrat, Diacetat, Difumarat, Dimeglumin, Diphosphat, Dinatrium und Trihydrochlorid, was jedoch keine Einschränkung darstellen soll.

Im Hinblick auf das oben Gesagte sieht man, daß unter dem Ausdruck "pharmazeutisch unbedenkliches Salz" im vorliegenden Zusammenhang ein Wirkstoff zu verstehen ist, der eine erfindungsgemäße Verbindung in der Form eines ihrer Salze enthält, insbesondere dann, wenn diese Salzform dem Wirkstoff im Vergleich zu der freien Form des Wirkstoffs oder irgendeiner anderen Salzform des Wirkstoffs, die früher verwendet wurde, verbesserte pharmakokinetische Eigenschaften verleiht. Die pharmazeutisch unbedenkliche Salzform des Wirkstoffs kann auch diesem Wirkstoff erst eine gewünschte pharmakokinetische Eigenschaft verleihen, über die er früher nicht verfügt hat, und kann sogar die Pharmakodynamik dieses Wirkstoffs in bezug auf seine therapeutische Wirksamkeit im Körper positiv beeinflussen.

Gegenstand der Erfindung sind ferner Arzneimittel, enthaltend mindestens eine erfindungsgemäße Verbindung und/oder ihre pharmazeutisch verwendbaren Derivate, Solvate und Stereoisomere, einschließlich deren Mischungen in allen Verhältnissen, sowie gegebenenfalls Träger- und/oder Hilfsstoffe.

Pharmazeutische Formulierungen können in Form von Dosiseinheiten, die eine vorbestimmte Menge an Wirkstoff pro Dosiseinheit enthalten, dargereicht werden. Eine solche Einheit kann beispielsweise 0,5 mg bis 1 g, vorzugsweise 1 mg bis 700 mg, besonders bevorzugt 5 mg bis 100 mg einer erfindungsgemäßen Verbindung enthalten, je nach dem behandelten Krankheitszustand, dem Verabreichungsweg und dem Alter, Gewicht und Zustand des Patienten, oder pharmazeutische Formulierungen können in Form von Dosiseinheiten, die eine vorbestimmte Menge an Wirkstoff pro Dosiseinheit enthalten, dargereicht werden. Bevorzugte Dosierungseinheitsformulierungen sind solche, die eine Tagesdosis oder Teildosis, wie oben angegeben, oder einen entsprechenden Bruchteil davon eines Wirkstoffs enthalten. Weiterhin lassen sich solche pharmazeutischen Formulierungen mit einem der im pharmazeutischen Fachgebiet allgemein bekannten Verfahren herstellen.

Pharmazeutische Formulierungen lassen sich zur Verabreichung über einen beliebigen geeigneten Weg, beispielsweise auf oralem (einschließlich buccalem bzw. sublingualem), rektalem, nasalem, topischem (einschließlich buccalem, sublingualem oder transdermalem), vaginalem oder parenteralem (einschließlich subkutanem, intramuskulärem, intravenösem oder intradermalem) Wege, anpassen. Solche Formulierungen können mit allen im pharmazeutischen Fachgebiet bekannten Verfahren hergestellt werden, indem beispielsweise der Wirkstoff mit dem bzw. den Trägerstoff(en) oder Hilfsstoff(en) zusammengebracht wird.

An die orale Verabreichung angepaßte pharmazeutische Formulierungen können als separate Einheiten, wie z.B. Kapseln oder Tabletten; Pulver oder Granulate; Lösungen oder Suspensionen in wäßrigen oder nichtwäßrigen Flüssigkeiten; eßbare Schäume oder Schaumspeisen; oder ÖI-in-Wasser-Flüssigemulsionen oder Wasser-in-ÖI-Flüssigemulsionen dargereicht werden.

So läßt sich beispielsweise bei der oralen Verabreichung in Form einer Tablette oder Kapsel die Wirkstoffkomponente mit einem oralen, nichttoxischen und pharmazeutisch unbedenklichen inerten Trägerstoff, wie z.B. Ethanol, Glyzerin, Wasser u.ä. kombinieren. Pulver werden hergestellt, indem die Verbindung auf eine geeignete feine Größe zerkleinert und mit einem in ähnlicher Weise zerkleinerten pharmazeutischen Trägerstoff, wie z.B. einem eßbaren Kohlenhydrat wie beispielsweise Stärke oder Mannit vermischt wird. Ein Geschmacksstoff, Konservierungsmittel, Dispersionsmittel und Farbstoff können ebenfalls vorhanden sein.

Kapseln werden hergestellt, indem ein Pulvergemisch wie oben beschrieben hergestellt und geformte Gelatinehüllen damit gefüllt werden. Gleit- und Schmiermittel wie z.B. hochdisperse Kieselsäure, Talkum, Magnesiumstearat, Kalziumstearat oder Polyethylenglykol in Festform können dem Pulvergemisch vor dem Füllvorgang zugesetzt werden. Ein Sprengmittel oder Lösungsvermittler, wie z.B. Agar-Agar, Kalziumcarbonat oder Natriumcarbonat, kann ebenfalls zugesetzt werden, um die Verfügbarkeit des Medikaments nach Einnahme der Kapsel zu verbessern. Außerdem können, falls gewünscht oder notwendig, geeignete Bindungs-, Schmier- und Sprengmittel sowie Farbstoffe ebenfalls in das Gemisch eingearbeitet werden. Zu den geeigneten Bindemitteln gehören Stärke, Gelatine, natürliche Zucker, wie z.B. Glukose oder Beta-Lactose, Süßstoffe aus Mais, natürliche und synthetische Gummi, wie z.B. Akazia, Traganth oder Natriumalginat, Carboxymethylzellulose, Polyethylenglykol, Wachse, u.ä. Zu den in diesen Dosierungsformen verwendeten Schmiermitteln gehören Natriumoleat, Natriumstearat, Magnesiumstearat, Natriumbenzoat, Natriumacetat, Natriumchlorid u.ä. Zu den Sprengmitteln gehören, ohne darauf beschränkt zu sein, Stärke, Methylzellulose, Agar, Bentonit, Xanthangummi u.ä. Die Tabletten werden formuliert, indem beispielsweise ein Pulvergemisch hergestellt, granuliert oder trockenverpreßt wird, ein Schmiermittel und ein Sprengmittel zugegeben werden und das Ganze zu Tabletten verpreßt wird. Ein Pulvergemisch wird hergestellt, indem die in geeigneter Weise zerkleinerte Verbindung mit einem Verdünnungsmittel oder einer Base, wie oben beschrieben, und gegebenenfalls mit einem Bindemittel, wie z.B. Carboxymethylzellulose, einem Alginat, Gelatine oder Polyvinylpyrrolidon, einem Lösungsverlangsamer, wie z.B. Paraffin, einem Resorptionsbeschleuniger, wie z.B. einem quaternären Salz und/oder einem Absorptionsmittel, wie z.B. Bentonit, Kaolin oder Dikalziumphosphat, vermischt wird. Das Pulvergemisch läßt sich granulieren, indem es mit einem Bindemittel, wie z.B. Sirup, Stärkepaste, Acadia-Schleim oder Lösungen aus Zellulose- oder Polymermaterialen benetzt und durch ein Sieb gepreßt wird. Als Alternative zur Granulierung kann man das Pulvergemisch durch eine Tablettiermaschine laufen lassen, wobei ungleichmäßig geformte Klumpen entstehen, die in Granulate aufgebrochen werden. Die Granulate können mittels Zugabe von Stearinsäure, einem Stearatsalz, Talkum oder Mineralöl gefettet werden, um ein Kleben an den Tablettengußformen zu verhindern. Das gefettete Gemisch wird dann zu Tabletten verpreßt. Die erfindungsgemäßen Verbindungen können auch mit einem freifließenden inerten Trägerstoff kombiniert und dann ohne Durchführung der Granulierungs-oder Trockenverpressungsschritte direkt zu Tabletten verpreßt werden. Eine durchsichtige oder undurchsichtige Schutzschicht, bestehend aus einer Versiegelung aus Schellack, einer Schicht aus Zucker oder Polymermaterial und einer Glanzschicht aus Wachs, kann vorhanden sein. Diesen Beschichtungen können Farbstoffe zugesetzt werden, um zwischen unterschiedlichen Dosierungseinheiten unterscheiden zu können.

Orale Flüssigkeiten, wie z.B. Lösung, Sirupe und Elixiere, können in Form von Dosierungseinheiten hergestellt werden, so daß eine gegebene Quantität eine vorgegebene Menge der Verbindung enthält. Sirupe lassen sich herstellen, indem die Verbindung in einer wäßrigen Lösung mit geeignetem Geschmack gelöst wird, während Elixiere unter Verwendung eines nichttoxischen alkoholischen Vehikels hergestellt werden. Suspensionen können durch Dispersion der Verbindung in einem nichttoxischen Vehikel formuliert werden. Lösungsvermittler und Emulgiermittel, wie z.B. ethoxylierte lsostearylalkohole und Polyoxyethylensorbitolether, Konservierungsmittel, Geschmackszusätze, wie z.B. Pfefferminzöl oder natürliche Süßstoffe oder Saccharin oder andere künstliche Süßstoffe, u.ä. können ebenfalls zugegeben werden.

Die Dosierungseinheitsformulierungen für die orale Verabreichung können gegebenenfalls in Mikrokapseln eingeschlossen werden. Die Formulierung läßt sich auch so herstellen, daß die Freisetzung verlängert oder retardiert wird, wie beispielsweise durch Beschichtung oder Einbettung von partikulärem Material in Polymere, Wachs u.ä.

Die erfindungsgemäßen Verbindungen sowie Salze, Solvate und physiologisch funktionelle Derivate davon lassen sich auch in Form von Liposomenzuführsystemen, wie z.B. kleinen unilamellaren Vesikeln, großen unilamellaren Vesikeln und multilamellaren Vesikeln, verabreichen. Liposomen können aus verschiedenen Phospholipiden, wie z.B. Cholesterin, Stearylamin oder Phosphatidylcholinen, gebildet werden.

Die erfindungsgemäßen Verbindungen sowie die Salze, Solvate und physiologisch funktionellen Derivate davon können auch unter Verwendung monoklonaler Antikörper als individuelle Träger, an die die Verbindungsmoleküle gekoppelt werden, zugeführt werden. Die Verbindungen können auch mit löslichen Polymeren als zielgerichtete Arzneistoffträger gekoppelt werden. Solche Polymere können Polyvinylpyrrolidon, Pyran-Copolymer, Polyhydroxypropylmethacrylamidphenol, Polyhydroxyethylaspartamidphenol oder Polyethylenoxidpolylysin, substituiert mit Palmitoylresten, umfassen. Weiterhin können die Verbindungen an eine Klasse von biologisch abbaubaren Polymeren, die zur Erzielung einer kontrollierten Freisetzung eines Arzneistoffs geeignet sind, z.B. Polymilchsäure, Polyepsilon-Caprolacton, Polyhydroxybuttersäure, Polyorthoester, Polyacetale, Polydihydroxypyrane, Polycyanoacrylate und quervernetzte oder amphipatische Blockcopolymere von Hydrogelen, gekoppelt sein.

An die transdermale Verabreichung angepaßte pharmazeutische Formulierungen können als eigenständige Pflaster für längeren, engen Kontakt mit der Epidermis des Empfängers dargereicht werden. So kann beispielsweise der Wirkstoff aus dem Pflaster mittels lontophorese zugeführt werden, wie in Pharmaceutical Research, 3(6), 318 (1986) allgemein beschrieben.

An die topische Verabreichung angepaßte pharmazeutische Verbindungen können als Salben, Cremes, Suspensionen, Lotionen, Pulver, Lösungen, Pasten, Gele, Sprays, Aerosole oder Öle formuliert sein.

Für Behandlungen des Auges oder anderer äußerer Gewebe, z.B. Mund und Haut, werden die Formulierungen vorzugsweise als topische Salbe oder Creme appliziert. Bei Formulierung zu einer Salbe kann der Wirkstoff entweder mit einer paraffinischen oder einer mit Wasser mischbaren Cremebasis eingesetzt werden. Alternativ kann der Wirkstoff zu einer Creme mit einer Öl-in-Wasser-Cremebasis oder einer Wasser-in-Öl-Basis formuliert werden.

Zu den an die topische Applikation am Auge angepaßten pharmazeutischen Formulierungen gehören Augentropfen, wobei der Wirkstoff in einem geeigneten Träger, insbesondere einem wäßrigen Lösungsmittel, gelöst oder suspendiert ist.

An die topische Applikation im Mund angepaßte pharmazeutische Formulierungen umfassen Lutschtabletten, Pastillen und Mundspülmittel.

An die rektale Verabreichung angepaßte pharmazeutische Formulierungen können in Form von Zäpfchen oder Einläufen dargereicht werden.

An die nasale Verabreichung angepaßte pharmazeutische Formulierungen, in denen die Trägersubstanz ein Feststoff ist, enthalten ein grobes Pulver mit einer Teilchengröße beispielsweise im Bereich von 20-500 Mikrometern, das in der Art und Weise, wie Schnupftabak aufgenommen wird, verabreicht wird, d.h. durch Schnellinhalation über die Nasenwege aus einem dicht an die Nase gehaltenen Behälter mit dem Pulver. Geeignete Formulierungen zur Verabreichung als Nasenspray oder Nasentropfen mit einer Flüssigkeit als Trägersubstanz umfassen Wirkstofflösungen in Wasser oder Öl.

An die Verabreichung durch Inhalation angepaßte pharmazeutische Formulierungen umfassen feinpartikuläre Stäube oder Nebel, die mittels verschiedener Arten von unter Druck stehenden Dosierspendern mit Aerosolen, Verneblern oder Insufflatoren erzeugt werden können.

An die vaginale Verabreichung angepaßte pharmazeutische Formulierungen können als Pessare, Tampons, Cremes, Gele, Pasten, Schäume oder Sprayformulierungen dargereicht werden.

Zu den an die parenterale Verabreichung angepaßten pharmazeutischen Formulierungen gehören wäßrige und nichtwäßrige sterile Injektionslösungen, die Antioxidantien, Puffer, Bakteriostatika und Solute, durch die die Formulierung isotonisch mit dem Blut des zu behandelnden Empfängers gemacht wird, enthalten; sowie wäßrige und nichtwäßrige sterile Suspensionen, die Suspensionsmittel und Verdicker enthalten können. Die Formulierungen können in Einzeldosis- oder Mehrfachdosisbehältern, z.B. versiegelten Ampullen und Fläschchen, dargereicht und in gefriergetrocknetem (lyophilisiertem) Zustand gelagert werden, so daß nur die Zugabe der sterilen Trägerflüssigkeit, z.B. Wasser für Injektionszwecke, unmittelbar vor Gebrauch erforderlich ist. Rezepturmäßig hergestellte Injektionslösungen und Suspensionen können aus sterilen Pulvern, Granulaten und Tabletten hergestellt werden.

Es versteht sich, daß die Formulierungen neben den obigen besonders erwähnten Bestandteilen andere im Fachgebiet übliche Mittel mit Bezug auf die jeweilige Art der Formulierung enthalten können; so können beispielsweise für die orale Verabreichung geeignete Formulierungen Geschmacksstoffe enthalten.

Eine therapeutisch wirksame Menge einer erfindungsgemäßen Verbindung hängt von einer Reihe von Faktoren ab, einschließlich z.B. dem Alter und Gewicht des Tiers, dem exakten Krankheitszustand, der der Behandlung bedarf, sowie seines Schweregrads, der Beschaffenheit der Formulierung sowie dem Verabreichungsweg, und wird letztendlich von dem behandelnden Arzt bzw. Tierarzt festgelegt. Jedoch liegt eine wirksame Menge einer erfindungsgemäßen Verbindung für die Behandlung von neoplastischem Wachstum, z.B. Dickdarm- oder Brustkarzinom, im allgemeinen im Bereich von 0,1 bis 100 mg/kg Körpergewicht des Empfängers (Säugers) pro Tag und besonders typisch im Bereich von 1 bis 10 mg/kg Körpergewicht pro Tag. Somit läge für einen 70 kg schweren erwachsenen Säuger die tatsächliche Menge pro Tag für gewöhnlich zwischen 70 und 700 mg, wobei diese Menge als Einzeldosis pro Tag oder üblicher in einer Reihe von Teildosen (wie z.B. zwei, drei, vier, fünf oder sechs) pro Tag gegeben werden kann, so daß die Gesamttagesdosis die gleiche ist. Eine wirksame Menge eines Salzes oder Solvats oder eines physiologisch funktionellen Derivats davon kann als Anteil der wirksamen Menge der erfindungsgemäßen Verbindung per se bestimmt werden. Es läßt sich annehmen, daß ähnliche Dosierungen für die Behandlung der anderen, obenerwähnten Krankheitszustände geeignet sind.

Gegenstand der Erfindung sind ferner Arzneimittel enthaltend mindestens eine erfindungsgemäße Verbindung und/oder ihre pharmazeutisch verwendbaren Derivate, Solvate und Stereoisomere, einschließlich deren Mischungen in allen Verhältnissen, und mindestens einen weiteren Arzneimittelwirkstoff.

Gegenstand der Erfindung ist auch ein Set (Kit), bestehend aus getrennten Packungen von
(a) einer wirksamen Menge an einer erfindungsgemäßen Verbindung und/oder ihrer pharmazeutisch verwendbaren Derivate, Solvate und Stereoisomere, einschließlich deren Mischungen in allen Verhältnissen,
   und
(b) einer wirksamen Menge eines weiteren Arzneimittelwirkstoffs.

Das Set enthält geeignete Behälter, wie Schachteln oder Kartons, individuelle Flaschen, Beutel oder Ampullen. Das Set kann z.B. separate Ampullen enthalten, in denen jeweils eine wirksame Menge an einer erfindungsgemäßen Verbindung und/oder ihrer pharmazeutisch verwendbaren Derivate, Solvate und Stereoisomere, einschließlich deren Mischungen in allen Verhältnissen,
und einer wirksamen Menge eines weiteren Arzneimittelwirkstoffs gelöst oder in lyophilisierter Form vorliegt.

### VERWENDUNG

Die erfindungsgemäßen Verbindungen sowie ihre pharmazeutisch verwendbaren Derivate, Salze, Solvate, Tautomeren und Stereoisomeren, einschließlich deren Mischungen in allen Verhältnissen, eignen sich als pharmazeutische Wirkstoffe für Säugetiere, insbesondere für den Menschen, zur Herstellung eines Arzneimittels zur Behandlung und/oder Bekämpfung von Krebs, Tumorwachstum, Metastasenwachstum, Fibrose, Restenose, HIV Infektion, Alzheimer, Atherosklerose und/oder zur Förderung der Wundheilung.

Insbesondere bevorzugt ist die Verwendung zur Behandlung einer Krankheit, wobei die Krankheit ein fester Tumor ist.
Der feste Tumor ist vorzugsweise ausgewählt aus der Gruppe der Tumoren des Plattenepithel, der Blasen, des Magens, der Nieren, von Kopf und Hals, des Ösophagus, des Gebärmutterhals, der Schilddrüse, des Darm, der Leber, des Gehirns, der Prostata, des Urogenitaltrakts, des lymphatischen Systems, des Magens, des Kehlkopf und/oder der Lunge.

Der feste Tumor ist weiterhin vorzugsweise ausgewählt aus der Gruppe Lungenadenokarzinom, kleinzellige Lungenkarzinome, Bauchspeicheldrüsenkrebs, Glioblastome, Kolonkarzinom und Brustkarzinom.

Weiterhin bevorzugt ist die Verwendung zur Behandlung eines Tumors des Blut- und Immunsystems, vorzugsweise zur Behandlung eines Tumors ausgewählt aus der Gruppe der akuten myelotischen Leukämie, der chronischen myelotischen Leukämie, akuten lymphatischen Leukämie und/oder chronischen lymphatischen Leukämie.

Die vorliegenden Verbindungen eignen sich auch zur Kombination mit bekannten Antikrebsmitteln. Zu diesen bekannten Antikrebsmitteln zählen die folgenden: Östrogenrezeptormodulatoren, Androgenrezeptormodulatoren, Retinoidrezeptormodulatoren, Zytotoxika, antiproliferative Mittel, Prenyl-Proteintransferasehemmer, HMG-CoA-Reduktase-Hemmer, HIV-Protease-Hemmer, Reverse-Transkriptase-Hemmer sowie weitere Angiogenesehemmer. Die vorliegenden Verbindungen eignen sich insbesondere zur gemeinsamen Anwendung mit Radiotherapie. Die synergistischen Wirkungen der Hemmung des VEGF in Kombination mit Radiotherapie sind in der Fachwelt beschrieben worden (siehe WO 00/61186).
"Östrogenrezeptormodulatoren" bezieht sich auf Verbindungen, die die Bindung von Östrogen an den Rezeptor stören oder diese hemmen, und zwar unabhängig davon, wie dies geschieht. Zu den Ostrogenrezeptormodulatoren zählen zum Beispiel Tamoxifen, Raloxifen, Idoxifen, LY353381, LY 117081, Toremifen, Fulvestrant, 4-[7-(2,2-Dimethyl-1-oxopropoxy-4-methyl-2-[4-[2-(1- piperidinyl)ethoxy]phenyl]-2H-1-benzopyran-3-yl]phenyl-2,2-dimethylpropanoat, 4,4'-Dihydroxybenzophenon-2,4-dinitrophenylhydrazon und SH646, was jedoch keine Einschränkung darstellen soll.
"Androgenrezeptormodulatoren" bezieht sich auf Verbindungen, die die Bindung von Androgenen an den Rezeptor stören oder diese hemmen, und zwar unabhängig davon, wie dies geschieht. Zu den Androgenrezeptormodulatoren zählen zum Beispiel Finasterid und andere 5α-Reduktase-Hemmer, Nilutamid, Flutamid, Bicalutamid, Liarozol und Abirateron-acetat.
"Retinoidrezeptormodulatoren" bezieht sich auf Verbindungen, die die Bindung von Retinoiden an den Rezeptor stören oder diese hemmen, und zwar unabhängig davon, wie dies geschieht. Zu solchen Retinoidrezeptormodulatoren zählen zum Beispiel Bexaroten, Tretinoin, 13-cis-Retinsäure, 9-cis-Retinsäure, α-Difluormethylornithin, ILX23-7553, trans-N-(4'-Hydroxyphenyl)retinamid und N-4-Carboxyphenylretinamid.
"Zytotoxika" bezieht sich auf Verbindungen, die in erster Linie durch direkte Einwirkung auf die Zellfunktion zum Zelltod führen oder die die Zellmyose hemmen oder diese stören, darunter Alkylierungsmittel, Tumornekrosefaktoren, interkaliernde Mittel, Mikrotubulin-Hemmer und Topoisomerase-Hemmer.
Zu den Zytotoxika zählen zum Beispiel Tirapazimin, Sertenef, Cachectin, Ifosfamid, Tasonermin, Lonidamin, Carboplatin, Altretamin, Prednimustin, Dibromdulcit, Ranimustin, Fotemustin, Nedaplatin, Oxaliplatin, Temozolomid, Heptaplatin, Estramustin, Improsulfan-tosylat, Trofosfamid, Nimustin, Dibrospidium-chlorid, Pumitepa, Lobaplatin, Satraplatin, Profiromycin, Cisplatin, Irofulven, Dexifosfamid, cis-Amindichlor(2-methylpyridin)platin, Benzylguanin, Glufosfamid, GPX100, (trans,trans,trans)-bis-mu-(hexan-1,6-diamin)-mu-[diamin-platin(II)]bis-[diamin(chlor)platin(II)]-tetrachlorid, Diarizidinylspermin, Arsentrioxid, 1-(11-Dodecylamino-10-hydroxyundecyl)-3,7-dimethylxanthin, Zorubicin, Idarubicin, Daunorubicin, Bisantren, Mitoxantron, Pirarubicin, Pinafid, Valrubicin, Amrubicin, Antineoplaston, 3'-Desamino-3'-morpholino-13-desoxo-10-hydroxycarminomycin, Annamycin, Galarubicin, Elinafid, MEN10755 und 4-Desmethoxy-3-desamino-3-aziridinyl-4-methylsulfonyl-daunorubicin (siehe WO 00/50032), was jedoch keine Einschränkung darstellen soll.
Zu den Mikrotubulin-Hemmern zählen zum Beispiel Paclitaxel, Vindesinsulfat, 3',4'-Dideshydro-4'-desoxy-8'-norvincaleukoblastin, Docetaxol, Rhizoxin, Dolastatin, Mivobulin-isethionat, Auristatin, Cemadotin, RPR109881, BMS184476, Vinflunin, Cryptophycin, 2,3,4,5,6-pentafluor-N-(3-fluor-4-methoxyphenyl)benzolsulfonamid, Anhydrovinblastin, N,N-dimethyl-L-valyl-L-valyl-N-methyl-L-valyl-L-prolyl-L-prolin-t-butylamid, TDX258 und BMS188797.

Topoisomerase-Hemmer sind zum Beispiel Topotecan, Hycaptamin, Irinotecan, Rubitecan, 6-Ethoxypropionyl-3',4'-O-exo-benzyliden-chartreusin, 9-Methoxy-N,N-dimethyl-5-nitropyrazolo[3,4,5-kl]acridin-2-(6H)propanamin, 1-Amino-9-ethyl-5-fluor-2,3-dihydro-9-hydroxy-4-methyl-1H,12H-benzo[de]pyrano[3',4':b,7]indolizino[1,2b]chinolin-10,13(9H,15H)-dion, Lurtotecan, 7-[2-(N-Isopropylamino)ethyl]-(20S)camptothecin, BNP1350, BNPI1100, BN80915, BN80942, Etoposid-phosphat, Teniposid, Sobuzoxan, 2'-Dimethylamino-2'-desoxy-etoposid, GL331, N-[2-(Dimethylamino)ethyl]-9-hydroxy-5,6-dimethyl-6H-pyrido[4,3-b]carbazol-1-carboxamid, Asulacrin, (5a,5aB,8aa,9b)-9-[2-[N-[2-(Dimethylamino)ethyl]-N-methylamino]ethyl]-5-[4-hydroxy-3,5-dimethoxyphenyl]-5,5a,6,8,8a,9-hexohydrofuro(3',4':6,7)naphtho(2,3-d)-1,3-dioxol-6-on, 2,3-(Methylendioxy)-5-methyl-7-hydroxy-8-methoxybenzo[c]phenanthridinium, 6,9-Bis[(2-aminoethyl)amino]benzo[g]isochinolin-5,10-dion, 5-(3-Aminopropylamino)-7,10-dihydroxy-2-(2-hydroxyethylaminomethyl)-6H-pyrazolo[4,5,1-de]-acridin-6-on, N-[1-[2(Diethylamino)ethylamino]-7-methoxy-9-oxo-9H-thioxanthen-4-ylmethyl]formamid, N-(2-(Dimethyl-amino)-ethyl)acridin-4-carboxamid, 6-[[2-(Dimethylamino)-ethyl]amino]-3-hydroxy-7H-indeno[2,1-c]chinolin-7-on und Dimesna.
Zu den "antiproliferativen Mitteln" zählen Antisense-RNA- und -DNA-Oligonucleotide wie G3139, ODN698, RVASKRAS, GEM231 und INX3001, sowie Antimetaboliten wie Enocitabin, Carmofur, Tegafur, Pentostatin, Doxifluridin, Trimetrexat, Fludarabin, Capecitabin, Galocitabin, Cytarabin-ocfosfat, Fosteabin-Natriumhydrat, Raltitrexed, Paltitrexid, Emitefur, Tiazofurin, Decitabin, Nolatrexed, Pemetrexed, Nelzarabin, 2'-Desoxy-2'-methylidencytidin, 2'-Fluormethylen-2'-desoxycytidin, N-[5-(2,3-Dihydrobenzofuryl)sulfonyl]-N'-(3,4-dichlorphenyl)harnstoff, N6-[4-Desoxy-4-[N2-[2(E),4(E)-tetradecadienoyl]glycylamino]-L-glycero-B-L-manno-heptopyranosyl]adenin, Aplidin, Ecteinascidin, Troxacitabine, 4-[2-Amino-4-oxo-4,6,7,8-tetrahydro-3H-pyrimidino[5,4-b][1,4]thiazin-6-yl-(S)-ethyl]-2,5-thienoyl-L-glutaminsäure, Aminopterin, 5-Flurouracil, Alanosin, 11-Acetyl-8-(carbamoyloxymethyl)-4-formyl-6-methoxy-14-oxa-1,11-diaza-tetracyclo(7.4.1.0.0)-tetradeca-2,4,6-trien-9-ylessigsäureester, Swainsonin, Lometrexol, Dexrazoxan, Methioninase, 2'-cyan-2'-desoxy-N4-palmitoyl-1-B-D-Arabinofuranosylcytosin und 3-Aminopyridin-2-carboxaldehyd-thiosemicarbazon. Die "antiproliferativen Mittel" beinhalten auch andere monoklonale Antikörper gegen Wachstumsfaktoren als bereits unter den "Angiogenese-Hemmern" angeführt wurden, wie Trastuzumab, sowie Tumorsuppressorgene, wie p53, die über rekombinanten virusvermittelten Gentransfer abgegeben werden können (siehe z.B. US-Patent Nr. 6,069,134).

### In vitro-(Enzym-)Assay zur Bestimmung der Wirksamkeit der Inhibitoren der Hemmung von TGF-beta vermittelten Wirkungen

Als Beispiel wird die Fähigkeit der Inhibitoren zur Aufhebung der TGF-beta vermittelten Wachstumshemmung getestet.
Zellen der Lungenepithelzellinie Mv1Lu werden in definierter Zelldichte in einer 96-well Mikrotiterplatte ausgesät und über Nacht unter Standardbedingungen kultiviert. Am Folgetag wird das Medium mit Medium, das 0,5%FCS und 1 ng/ml TGF-beta enthält, ersetzt und die Testsubstanzen in definierten Konzentrationen, in der Regel in Form von Verdünnungsreihen mit 5-fach Schritten, zugegeben. Die Konzentration des Lösungsmittels DMSO liegt konstant bei 0,5%. Nach zwei weiteren Tagen erfolgt Kristallviolett-Färbung der Zellen. Nach Extraktion des Kristallviolett aus den fixierten Zellen wird die Absorption bei 550 nm spektralphotometrisch gemessen. Sie kann als quantitatives Maß für die vorhandenen adhärenten Zellen und damit der Zellproliferation während der Kultur herangezogen werden.

**Tabelle 1: Inhibierung von TGF-beta**

| Verbindung Nr. | IC₅₀ [mol/l] |
|---|---|
| "A1" | 3.9 E-06 |
| "A2" | 0.2 E-06 |
| "A4" | 9.9 E-06 |
| "A18" | 3.3 E-06 |
| "A19" | 2.5 E-06 |
| | |
| | |

### Zellulärer Assay zur Testung von TGF-beta-Rezeptor-I-Kinase-Inhibitoren

Der Kinaseassay wird als 384-well Flashplate assay durchgeführt. 31.2 nM GST-ALK5, 439 nM GST-SMAD2 und 3 mM ATP (mit 0.3µCi ³³P-ATP/well) werden in einem Gesamtvolumen von 35µl (20 mM HEPES, 10 mM MgCl, 5 mM MnCl, 1 mM DTT, 0.1 % BSA, pH 7.4) ohne oder mit Prüfsubstanz (5-10 Konzentrationen) für 45 Min bei 30°C inkubiert. Die Reaktion wird mit 25µl 200 mM EDTA-Lösung gestoppt, nach 30 Min bei Raumtemperatur abgesaugt und die Wells mit 3mal 100 µl 0.9%-ige NaCl-Lösung gewaschen. Radioaktivität wird im Topcount gemessen. Die IC₅₀-Werte werden mit RS1 berechnet.

Vor- und nachstehend sind alle Temperaturen in °C angegeben. In den nachfolgenden Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und /oder durch Kristallisation. Rf-Werte an Kieselgel; Laufmittel: Ethylacetat/Methanol 9:1.

| | |
|---|---|
| Massenspektrometrie (MS): | EI (Elektronenstoß-lonisation) M⁺ |
| | FAB (Fast Atom Bombardment) (M+H)⁺ |
| | ESI (Electrospray Ionization) (M+H)⁺ |

APCI-MS (atmospheric pressure chemical ionization - mass spectrometry) (M+H)⁺.

### Retentionszeit Rₜ [min]: Bestimmung erfolgt mit HPLC

Säule: Chromolith SpeedROD, 50 x 4.6 mm² (Best.Nr. 1.51450.0001) von Merck
Gradient: 5.0 min, t = 0 min, A:B = 95:5, t = 4.4 min: A:B = 25:75,
t = 4.5 min bis t = 5.0 min: A:B = 0:100
Fluß: 3.00 ml/min
Laufmittel A: Wasser + 0, 1 % TFA (Trifluoressigsäure),
Laufmittel B: Acetonitril + 0,08% TFA
Wellenlänge: 220 nm

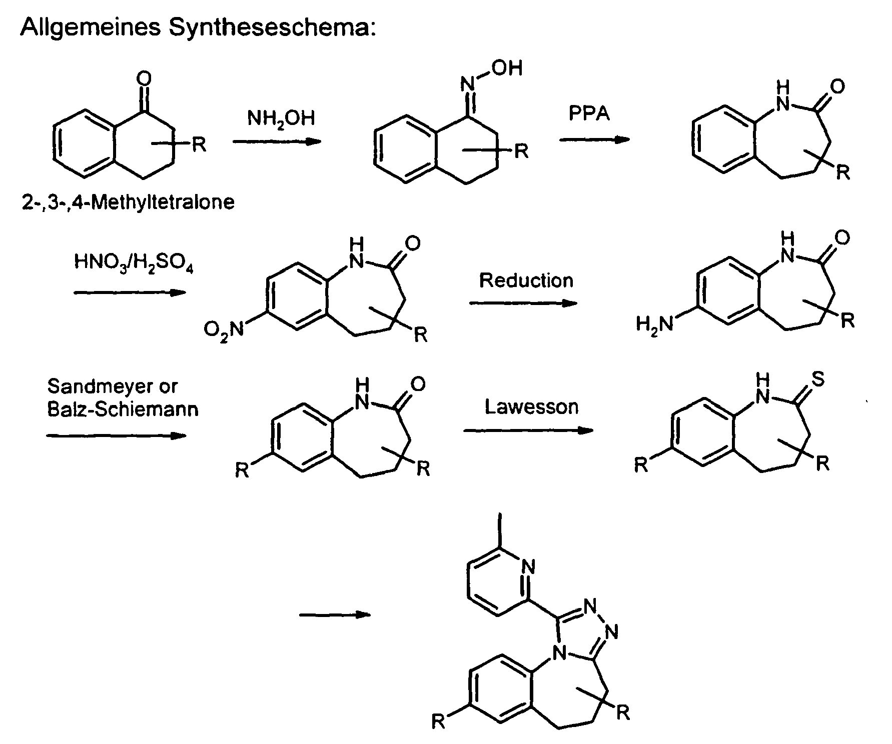

Anstelle der Nitrierung (HNO₃/H₂SO₄) ist auch Bromierung möglich mit anschließendem Austausch durch CN oder OMe (siehe Beispiele).

### Beispiel 1

### Herstellung von 8-Methoxy-4-methyl-1-(6-methyl-pyridin-2-yl)-5,6-dihydro-4H-2,3,10b-triaza-benz[e]azulen ("A1")

1.1 In einem, mit Stickstoff inertisierten, 100 ml Dreihalskolben werden 1,00 g Natrium in 10 ml Methanol gelöst. Die Mischung wird bis 35 °C erwärmt. 1,00 g 7-Brom-3-methyl-2,3,4,5-tetrahydro-1*H*-1-benzazepin-2-on [Herstellung nach K. Hino et al., Chem. Pharm. Bull. 36, 2386-2400, 1988] werden in 12 ml DMF gelöst. Nachdem das Natrium vollständig gelöst ist, wird das Benzazepinon zugegeben. Anschließend werden noch 1,47 g Kupfer(I)-iodid zugegeben. Das Reaktionsgemisch wird bis 120 °C (Ölbadtemp.) erhitzt (RF) und so über Nacht gerührt.
Zur Aufarbeitung wird die Reaktion bis RT gekühlt. Das Gemisch wird über eine mit Kieselgur belegte Fritte abgesaugt. Es wird gut mit DMF nachgewaschen. Das Filtrat wird am Hochvakuumrotavapor eingeengt. Der Rückstand wird in Dichlormethan aufgenommen und mit Natronlauge, w = 2 %, extrahiert. Die wässrige Phase wird mit DCM nachextrahiert. Die vereinten organischen Phasen werden mit konz. Kochsalzlösung gewaschen, über Natriumsulfat getrocknet, filtriert und zum Rückstand eingeengt.
Der Rückstand wird mit Petroleumbenzin verrieben, abgesaugt und gut nachgewaschen.
Ausbeute: 0,6610 g violette Kristalle 7-Methoxy-3-methyl-2,3,4,5-tetrahydro-1*H*-1-benzazepin-2-on.

1.2 In einem 100 ml Rundkolben werden 655 mg 7-Methoxy-3-methyl-2,3,4,5-tetrahydro-1*H*-1-benzazepin-2-on in 15 ml Toluol suspendiert. Man gibt 1,012 g 2,4-Bis-(4-phenoxy-phenyl)-1,3,2,4-dithiadiphosphetan-2,4-disulfid dazu.Die gelbe Suspension wird unter Rühren zum Rückfluß erhitzt und 1,5 h lang gekocht.
Der Ansatz wird anschließend auf Eiswasser gegossen und man arbeitet wie üblich auf.
Ausbeute: 1,5645 g 7-Methoxy-3-methyl-1,3,4,5-tetrahydro-1-benzazepin-2-thion.

1.3 In einem 100 ml Rundkolben werden 1,5645 g 7-Methoxy-3-methyl-1,3,4,5-tetrahydro-1-benzazepin-2-thion in 20 ml 1-Butanol gelöst. Man gibt 1,68 g 6-Methyl-pyridin-2-carbonsäure-hydrazid dazu. Das Reaktionsgemisch wird zum Rückfluß erhitzt und über Nacht bei 120 °C (Ölbadtemp.) gekocht.
Zur Aufarbeitung wird der Ansatz unter Rühren bis 40 °C abgekühlt und mit 50 ml Ethylacetat verdünnt. Die rotbraune Lösung wird dreimal mit je 20 ml halbkonz. Kochsalzlösung gewaschen. Die organische Phase wird über Natriumsulfat getrocknet, filtriert und zum Rückstand eingeengt.
Der Rückstand wird in Ethylacetat aufgenommen und zweimal mit 5 % iger Citronensäure extrahiert. Zuletzt wird der pH Wert der organischen Phase durch Ausschütteln mit halbkonz. Bicarbonatlösung wieder in den basischen Bereich verschoben. Die organische Phase wird über Natriumsulfat getrocknet, filtriert und zum Rückstand eingeengt. Der Rückstand wird über Kieselgel chromatographiert.

Säulenbedingungen:
Länge: 30 cm Durchmesser: 3,5 cm Füllung: Kieselgel 60 (0,04-0,063 mm)
Fließmittel: DCM/Methanol, 95:5
Man erhält 585 mg "A1", EI-MS [M⁺] 320, 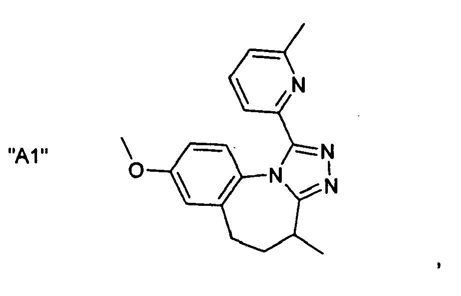 ¹H-NMR (500 MHz, DMSO-d₆) δ [ppm]: 7.8 (1H, t), 7.7 (1H,d), 7.3 (1H,d), 7.0 (1H,d), 6.95 (H,d), 6.8 (1H,dd), 3.85 (3H,s), 2.7 (3H,m), 2.4 (1H,m), 2.3 (3H,s), 1.9 (1H,m), 1.3 (3H,br).

### Beispiel 2

### Herstellung von 8-Hydroxy-4-methyl-1-(6-methyl-pyridin-2-yl)-5,6-dihydro-4H-2,3,10b-triaza-benz[e]azulen ("A2")

In einem 100 ml Rundkolben werden 400 mg 8-Methoxy-4-methyl-1-(6-methyl-pyridin-2-yl)-5,6-dihydro-4H-2,3,10b-triaza-benz[e]azulen in 15 ml getrocknetem Dichlormethan gelöst. Man gibt 0,56 ml Bortribromid dazu. Die Lösung wird 4 Stunden bei RT gerührt.
Dann wird der Ansatz auf ein Gemisch aus Eiswasser und konz. Bicarbonatlösung gegossen und man arbeitet wie üblich auf.
Der Rückstand wird per Flashchromatographie aufgereinigt. Säulenbedingungen:
Länge: 30 cm, Durchmesser: 3,5 cm, Füllung: Kieselgel 60 (0,04-0,063 mm)
Fließmittel: DCM/Methanol, 95:5 (2 L)

Man erhält 305 mg gelbliches Öl. Das Öl wird durch präparative HPLC gereinigt. Man erhält 119 mg "A2" als TFA-Salz.
Gehalt HPLC: 99,6 %; HPLC-MS [M+H⁺] 307;
¹H-NMR (500 MHz, DMSO-d₆) δ [ppm]: 9.7 (1H,s), 7.8 (1H, t), 7.7 (1H,d), 7.3 (1H,d), 6.9 (1H,d), 6.8 (1H,d), 6.6 (1H,dd), 2.7 (3H,m), 2.4 (1H,m), 2.3 (3H,s), 1.8 (1H,m), 1.3 (3H,br).

### Beispiel 3

### Herstellung von 8-Fluor-5-methyl-1-(6-methyl-pyridin-2-yl)-5,6-dihydro-4H-2,3,10b-triaza-benzo[e]azulen ("A3")

3.1 1,2884 g 4-Methyl-1,3,4,5-tetrahydro-1-benzazepin-2-on werden in Schwefelsäure (20 ml) gelöst und die Lösung auf -10°C abgekühlt. Nun gibt man langsam HNO₃ (1,6 ml) über einen Tropftrichter hinzu. Man läßt 15 min nachrühren.
Die Reaktionslösung wird auf Eiswasser gegeben und der entstehene Niederschlag abfiltriert und gut mit Wasser gewaschen.
Man erhält 1,3852 g 4-Methyl-7-nitro-1,3,4,5-tetrahydro-1-benzazepin-2-on.
Diese Substanz wird in Methanol gelöst und hydriert (Pd-C 5%).

Man erhält 770 mg 4-Methyl-7-amino-1,3,4,5-tetrahydro-1-benzazepin-2-on;
¹H-NMR (500 MHz, DMSO-d₆) δ [ppm]: 9.0 (1H,s), 6.6 (1H,d), 6.41 (1H,s), 6.39 (1H,d), 4.4 (2H,br), 2.7 (2H,m), 2.4 (1H,m), 2.1 (2H,m), 1.7 (1H,m), 1.0 (3H,d).

3.2 In einem 250 ml Dreihalskolben mit Tropftrichter, Thermometer und Trockenrohr werden 453 mg Nitrosyltetrafluoroborat in 40 ml getrocknetem Acetonitril gelöst. Die Lösung wird unter Rühren bis 0 °C eingekühlt. Jetzt wird die Suspension aus 0,76 g 4-Methyl-7-amino-1,3,4,5-tetrahydro-1-benzazepin-2-on in 20 ml getrocknetem Acetonitril zugetropft. Die Temperatur wird mit dem Kältebad zwischen - 2 und 0 °C gehalten. Es wird 45 Min. bei dieser Temperatur nachgerührt.

Zur Aufarbeitung des Diazoniumsalzes wird zur Lösung 100 ml getrockneter Diethylether gegeben. Es wird 30 Min. gerührt und läßt auf RT erwärmen. Die Ansatzlösung wird am Rotationsverdampfer aufkonzentriert.
Der erhaltene ölige Rückstand wird portionsweise in 60 ml siedendes Toluol gegeben. Nach der Zugabe wird weitere 15 Min. am Rückfluß gekocht.
Das Reaktionsgemisch wird auf RT abgekühlt und über Kieselgur (Celite) filtriert. Der Nutschkuchen wird mit mehreren Portionen Chloroform nachgewaschen. Das Filtrat wird zum Rückstand eingeengt. Das Rohprodukt wird per Flashchromatographie aufgereinigt. Säulenbedingungen:
Länge: 30 cm, Durchmessser: 3,5 cm, Füllung: Kieselgel 60 (0,04-0,063 mm)
Fließmittel: PE/EE, 7:3 (1 I)
Man erhält 144 mg 7-Fluor-4-methyl-1,3,4,5-tetrahydro-1-benzazepin-2-on; ¹H-NMR (500 MHz, DMSO-d₆) δ [ppm]: 7.1 (1H,dd), 7.05 (1H,m), 6.97 (1H,dd), 2.8 (1H,m), 2.45 (1H,m), 2.3 (2H,m), 2.2 (1H,m), 1.8 (1H,m), 1.0 (3H,d).

3.3 Ausgehend von 144 mg 7-Fluor-4-methyl-1,3,4,5-tetrahydro-1-benzazepin-2-on erhält man, analog Beispiel 1, nach Umsetzung mit dem modifizierten Lawesson-Reagenz und anschließender Reaktion zum Triazol, 32 mg 8-Fluor-5-methyl-1-(6-methyl-pyridin-2-yl)-5,6-dihydro-4*H-*2,3,10b-triaza-benzo[e]azulen ("A3"); EI-MS [M⁺] 308;
¹H-NMR (500 MHz, DMSO-d₆) δ [ppm]: 7.8 (2H,m), 7.3 (2H,m), 7.2 (2H,m), 3.1 (1H,br), 2.8 (3H,br), 2.2 (3H,s),1.9 (1H,br), 1.0 (3H,d).

### Beispiel 4

### Herstellung von 8-Chlor-6-methyl-1-(6-methyl-pyridin-2-yl)-5,6-dihydro-4H-2,3,10b-triaza-benzo[e]azulen ("A4")

2.9 g 5-Methyl-1,3,4,5-tetrahydro-1-benzazepin-2-on werden in Analogie zu Beispiel 3.1 nitriert. Nach Aufreinigung durch Kieselgelchromatographie erhält man 1.9 g 5-Methyl-7-nitro-1,3,4,5-tetrahydro-1-benzazepin-2-on; ¹H-NMR (500 MHz, DMSO-d₆) δ [ppm]: 10.1 (1H, s), 8.13 (1H,dd), 8.07 (1H, d), 7.2 (1H,d), 3.1 (1H,m), 2.2 (3H,s), 1.8 (1H,m), 1.4 (3H,d).

Die Substanz wird hydriert zu 5-Methyl-7-amino-1,3,4,5-tetrahydro-1-benzazepin-2-on.

1,2 g der Aminoverbindung werden in 30 ml Salzsäure gelöst und auf 0°C abgekühlt. Bei 0 bis 5°C gibt man eine kalte Lösung aus Natriumnitrit (439,1 mg) in Wasser tropfenweise hinzu. Es bildet sich eine braune Lösung. Nach beendigter Zugabe läßt man noch 30 min bei ca. 0°C weiterrühren. Zur hergestellten Diazoniumlösung wird bei 0°C tropfenweise eine kalte Lösung aus Cu(I)-Chlorid (873,5 mg) in 20 ml Salzsäure zugegeben. Nach beendeter Zugabe läßt man langsam auf RT erwärmen. Zur Aufarbeitung wird die Reaktionslösung in 100 ml Wasser aufgenommen und 3x mit Essigester gewaschen. Die vereinigten orgaischen Phasen werden 2x mit 1 N HCl und mit Wasser gewaschen. Man trocknet über Natriumsulfat und entfernt das Lösemittel am Rotationsverdampfer.
Es werden 780 mg 7-Chlor-5-methyl-1,3,4,5-tetrahydro-1-benzazepin-2-on erhalten.

Die weitere Umsetzung von 250 mg des Produktes mit dem modifizierten Lawesson-Reagenz und anschließender Umsetzung mit 6-Methyl-pyridin-2-carbonsäure-hydrazid ergibt 80 mg 8-Chlor-6-methyl-1-(6-methyl-pyridin-2-yl)-5,6-dihydro-4*H*-2,3,10b-triaza-benzo[e]azulen, Trifluoracetat ("A4"); HPLC-MS [M+H⁺] 325.

Analog den vorstehenden Beispielen werden die nachstehenden Verbindungen erhalten

| Nr. | Name und/oder Struktur | MS |
|---|---|---|
| "A5" | 8-Hydroxy-6-methyl-1-(6-methyl-pyridin-2-yl)-5,6-dihydro-4*H*-2,3,10b-triaza-benzo[e]azulen, Trifluoracetat | EI-MS [M⁺] 306 |
| | 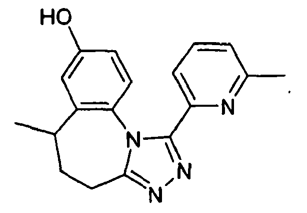 | |
| | ¹H-NMR (500 MHz, DMSO-d₆) δ [ppm]: 9.7 (1H,s), 7.9 (1H, t), 7.6 (1H,d), 7.55 (1H,d), 7.1 (1H,d), 6.9 (1H,d), 6.7 (1H,dd), 3.2 (1H,m), 2.9 (1H,m), 2.5 (1H,m), 2.45 (3H,s), 2.4 (1H,m), 1.9 (1H,m), 1.3 (3H,br) | |
| "A6" | 1-(6-Methyl-pyridin-2-yl)-5,6-dihydro-4*H*-2,3,10b-triaza-benzo[e]azulen, Trifluoracetat | EI-MS [M⁺] 276 |
| | 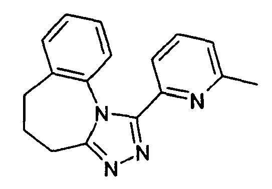 | |
| "A7" | 8-Brom-4-methyl-1-(6-methyl-pyridin-2-yl)-5,6-dihydro-4*H*-2,3,10b-triaza-benzo[e]azulen | EI-MS [M⁺] 369 |
| | 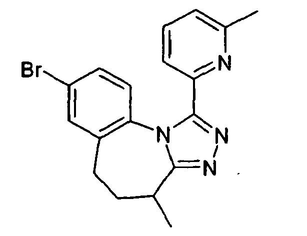 | |
| "A8" | 4-Methyl-1-(6-methyl-pyridin-2-yl)-5,6-dihydro-4H-2,3,10b-triaza-benzo[e]azulen, Bis-Trifluoracetat | EI-MS [M⁺] 290 |
| "A9" | 6-Methyl-1-(6-methyl-pyridin-2-yl)-5,6-dihydro-4H-2,3,10b-triaza-benzo[e]azulene | HPLC-MS [M+H⁺] 291 |
| "A10" | 8-Brom-1-(6-methyl-pyridin-2-yl)-5,6-dihydro-4*H*-2,3,10b-triaza-benzo[e]azulen, Trifluoracetat | HPLC-MS [M+H⁺] 356 |
| "A11" | 8-Brom-5-methyl-1-(6-methyl-pyridin-2-yl)-5,6-dihydro-4*H*-2,3,10b-triaza-benzo[e]azulen | HPLC-MS [M+H⁺] 370 |
| "A12" | 7-Methoxy-1-(6-methyl-pyridin-2-yl)-5,6-dihydro-4*H*-2,3,10b-triaza-benzo[e]azulen | EI-MS [M⁺] 306 |
| "A13" | 7-Hydroxy-1-(6-methyl-pyridin-2-yl)-5,6-dihydro-4*H*-2,3,10b-triaza-benzo[e]azulen | HPLC-MS [M+H⁺] 293 |
| "A14" | 8-Cyan-4-methyl-1-(6-methyl-pyridin-2-yl)-5,6-dihydro-4*H*-2,3,10b-triaza-benzo[e]azulen, Trifluoracetat | EI-MS [M⁺] 315 |
| "A15" | 8-Cyan-5-methyl-1-(6-methyl-pyridin-2-yl)-5,6-dihydro-4*H*-2,3,10b-triaza-benzo[e]azulen, Trifluoracetat | HPLC-MS [M+H⁺] 316 |
| "A16" | 7-Acetoxy-1-(6-methyl-pyridin-2-yl)-5,6-dihydro-4*H*-2,3,10b-triaza-benzo[e]azulen | HPLC-MS [M+H⁺] 335 |
| "A17" | 8-Chlor-5-methyl-1-(6-methyl-pyridin-2-yl)-5,6-dihydro-4*H*-2,3,10b-triaza-benzo[e]azulen | HPLC-MS [M+H⁺] 325 |
| "A18" | 8-Methoxy-6-methyl-1-(6-methyl-pyridin-2-yl)-5,6-dihydro-4*H*-2,3,10b-triaza-benzo[e]azulen, Trifluoracetat | HPLC-MS [M+H⁺] 321 |
| "A19" | 8-Hydroxy-4-ethyl-1-(6-methyl-pyridin-2-yl)-5,6-dihydro-4*H*-2,3,10b-triaza-benzo[e]azulen | |
| "A20" | 8-Hydroxy-4-propyl-1-(6-methyl-pyridin-2-yl)-5,6-dihydro-4*H*-2,3,10b-triaza-benzo[e]azulen | |
| "A21" | 8-Hydroxy-4-isobutyl-1-(6-methyl-pyridin-2-yl)-5,6-dihydro-4*H*-2,3,10b-triaza-benzo[e]azulen | |
| | | |

Die nachfolgenden Beispiele betreffen Arzneimittel:

### Beispiel A: Injektionsgläser

Eine Lösung von 100 g eines Wirkstoffes der Formel I und 5 g Dinatriumhydrogenphosphat wird in 3 I zweifach destilliertem Wasser mit 2 N Salzsäure auf pH 6,5 eingestellt; steril filtriert, in Injektionsgläser abgefüllt, unter sterilen Bedingungen lyophilisiert und steril verschlossen. Jedes Injektionsglas enthält 5 mg Wirkstoff.

### Beispiel B: Suppositorien

Man schmilzt ein Gemisch von 20 g eines Wirkstoffes der Formel I mit 100 g Sojalecithin und 1400 g Kakaobutter, gießt in Formen und läßt erkalten. Jedes Suppositorium enthält 20 mg Wirkstoff.

### Beispiel C: Lösung

Man bereitet eine Lösung aus 1 g eines Wirkstoffes der Formel I, 9,38 g NaH₂PO₄ · 2 H₂O, 28,48 g Na₂HPO₄ · 12 H₂O und 0,1 g Benzalkoniumchlorid in 940 ml zweifach destilliertem Wasser. Man stellt auf pH 6,8 ein, füllt auf 1 I auf und sterilisiert durch Bestrahlung. Diese Lösung kann in Form von Augentropfen verwendet werden.

### Beispiel D: Salbe

Man mischt 500 mg eines Wirkstoffes der Formel I mit 99,5 g Vaseline unter aseptischen Bedingungen.

### Beispiel E: Tabletten

Ein Gemisch von 1 kg Wirkstoff der Formel I, 4 kg Lactose, 1,2 kg Kartoffelstärke, 0,2 kg Talk und 0,1 kg Magnesiumstearat wird in üblicher Weise zu Tabletten verpreßt, derart, daß jede Tablette 10 mg Wirkstoff enthält.

### Beispiel F: Dragees

Analog Beispiel E werden Tabletten gepreßt, die anschließend in üblicher Weise mit einem Überzug aus Saccharose, Kartoffelstärke, Talk, Tragant und Farbstoff überzogen werden.

### Beispiel G: Kapseln

2 kg Wirkstoff der Formel I werden in üblicher Weise in Hartgelatinekapseln gefüllt, so daß jede Kapsel 20 mg des Wirkstoffs enthält.

### Beispiel H: Ampullen

Eine Lösung von 1 kg Wirkstoff der Formel I in 60 I zweifach destilliertem Wasser wird steril filtriert, in Ampullen abgefüllt, unter sterilen Bedingungen lyophilisiert und steril verschlossen. Jede Ampulle enthält 10 mg Wirkstoff.

## Patentansprüche

1. Verbindungen der Formel I 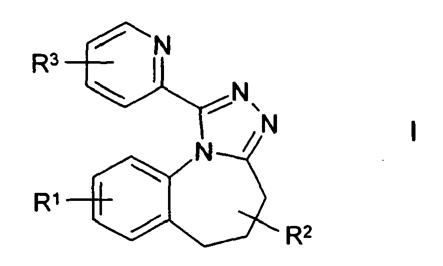 worin
R¹ H, A, OH, OA, NO₂, NH₂, NHA, NA₂, Hal, CN, A-COO, COOH, COOA oder CONR⁴R⁵,
R², R³ jeweils unabhängig voneinander H, A, Alkenyl mit 2-6 C- Atomen, Alkinyl mit 2-6 C-Atomen oder Hal,
R⁴, R⁵ jeweils unabhängig voneinander H oder A,
A unverzweigtes oder verzweigtes Alkyl mit 1, 2, 3, 4, 5, 6, 7, 8, 9 oder 10 C-Atomen, worin 1-7 H-Atome durch F ersetzt sein können,
Hal F, Cl, Br oder I,
bedeuten,
sowie ihre pharmazeutisch verwendbaren Derivate, Solvate, Salze, Tautomere und Stereoisomere, einschließlich deren Mischungen in allen Verhältnissen.

2. Verbindungen nach Anspruch 1, worin
R¹ H, OH, OA, Hal, CN oder A-COO,
bedeutet,
sowie ihre pharmazeutisch verwendbaren Derivate, Solvate, Salze und Stereoisomere, einschließlich deren Mischungen in allen Verhältnissen.

3. Verbindungen nach Anspruch 1 oder 2, worin
R² H oder A bedeutet,
sowie ihre pharmazeutisch verwendbaren Derivate, Solvate, Salze und Stereoisomere, einschließlich deren Mischungen in allen Verhältnissen.

4. Verbindungen nach Anspruch 1, 2 oder 3, worin
R³ A bedeutet,
sowie ihre pharmazeutisch verwendbaren Derivate, Solvate, Salze und Stereoisomere, einschließlich deren Mischungen in allen Verhältnissen.

5. Verbindungen gemäß einem oder mehreren der Ansprüche 1-4, worin
A unverzweigtes oder verzweigtes Alkyl mit 1, 2, 3, 4, 5 oder 6 C-Atomen, worin 1-5 H-Atome durch F ersetzt sein können,
sowie ihre pharmazeutisch verwendbaren Derivate, Solvate, Salze und Stereoisomere, einschließlich deren Mischungen in allen Verhältnissen.

6. Verbindungen gemäß einem oder mehreren der Ansprüche 1-5, worin
R¹ H, OH, OA, Hal, CN oder A-COO,
R² H oder A,
R³ A,
A unverzweigtes oder verzweigtes Alkyl mit 1, 2, 3, 4, 5 oder 6 C-Atomen, worin 1-5 H-Atome durch F ersetzt sein können,
Hal F, Cl, Br oder I,
bedeuten,
sowie ihre pharmazeutisch verwendbaren Derivate, Solvate, Salze und Stereoisomere, einschließlich deren Mischungen in allen Verhältnissen.

7. Verbindungen nach Anspruch 1 ausgewählt aus der Gruppe
| Nr. | Name und/oder Struktur |
|---|---|
| "A1" | 8-Methoxy-4-methyl-1-(6-methyl-pyridin-2-yl)-5,6-dihydro-4*H*-2,3,10b-triaza-benz[e]azulen |
| "A2" | 8-Hydroxy-4-methyl-1-(6-methyl-pyridin-2-yl)-5,6-dihydro-4*H*-2,3,10b-triaza-benz[e]azulen |
| "A3" | 8-Fluor-5-methyl-1-(6-methyl-pyridin-2-yl)-5,6-dihydro-4*H*-2,3,10b-triaza-benzo[e]azulen |
| "A4" | 8-Chlor-6-methyl-1-(6-methyl-pyridin-2-yl)-5,6-dihydro-4*H*-2,3,10b-triaza-benzo[e]azulen |
| "A5" | 8-Hydroxy-6-methyl-1-(6-methyl-pyridin-2-yl)-5,6-dihydro-4*H*-2,3,10b-triaza-benzo[e]azulen, Trifluoracetat |
| | 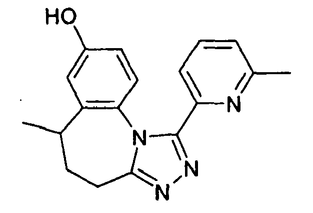 |
| "A6" | 1-(6-Methyl-pyridin-2-yl)-5,6-dihydro-4*H*-2,3,10b-triaza-benzo[e]azulen, Trifluoracetat |
| | 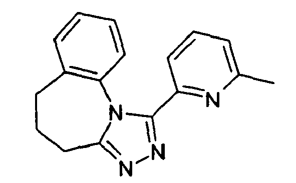 |
| "A7" | 8-Brom-4-methyl-1-(6-methyl-pyridin-2-yl)-5,6-dihydro-4*H*-2,3,10b-triaza-benzo[e]azulen |
| | 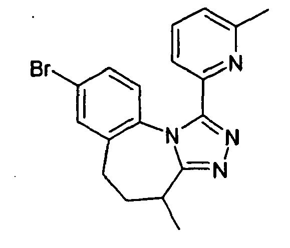 |
| "A8" | 4-Methyl-1-(6-methyl-pyridin-2-yl)-5,6-dihydro-4*H*-2,3,10b-triaza-benzo[e]azulen, Bis-Trifluoracetat |
| "A9" | 6-Methyl-1-(6-methyl-pyridin-2-yl)-5,6-dihydro-4*H*-2,3,10b-triaza-benzo[e]azulene |
| "A10" | 8-Brom-1-(6-methyl-pyridin-2-yl)-5,6-dihydro-4*H*-2,3,10b-triaza-benzo[e]azulen, Trifluoracetat |
| "A11" | 8-Brom-5-methyl-1-(6-methyl-pyridin-2-yl)-5,6-dihydro-4*H*-2,3,10b-triaza-benzo[e]azulen |
| "A12" | 7-Methoxy-1-(6-methyl-pyridin-2-yl)-5,6-dihydro-4*H*-2,3,10b-triaza-benzo[e]azulen |
| "A13" | 7-Hydroxy-1-(6-methyl-pyridin-2-yl)-5,6-dihydro-4*H-*2,3,10b-triaza-benzo[e]azulen |
| "A14" | 8-Cyan-4-methyl-1-(6-methyl-pyridin-2-yl)-5,6-dihydro-4*H*-2,3,10b-triaza-benzo[e]azulen, Trifluoracetat |
| "A15" | 8-Cyan-5-methyl-1-(6-methyl-pyridin-2-yl)-5,6-dihydro-4*H*-2,3,10b-triaza-benzo[e]azulen, Trifluoracetat |
| "A16" | 7-Acetoxy-1-(6-methyl-pyridin-2-yl)-5,6-dihydro-4*H*-2,3,10b-triaza-benzo[e]azulen |
| "A17" | 8-Chlor-5-methyl-1-(6-methyl-pyridin-2-yl)-5,6-dihydro-4*H*-2,3,10b-triaza-benzo[e]azulen |
| "A18" | 8-Methoxy-6-methyl-1-(6-methyl-pyridin-2-yl)-5,6-dihydro-4*H*-2,3,10b-triaza-benzo[e]azulen, Trifluoracetat |
| "A19" | 8-Hydroxy-4-ethyl-1-(6-methyl-pyridin-2-yl)-5,6-dihydro-4*H*-2,3,10b-triaza-benzo[e]azulen |
| "A20" | 8-Hydroxy-4-propyl-1-(6-methyl-pyridin-2-yl)-5,6-dihydro-4*H*-2,3,10b-triaza-benzo[e]azulen |
| "A21" | 8-Hydroxy-4-isobutyl-1-(6-methyl-pyridin-2-yl)-5,6-dihydro-4*H*-2,3,10b-triaza-benzo[e]azulen |
| | |
sowie ihre pharmazeutisch verwendbaren Derivate, Solvate, Salze und Stereoisomere, einschließlich deren Mischungen in allen Verhältnissen.

8. Verfahren zur Herstellung von Verbindungen der Formel I nach den Ansprüchen 1-7 sowie ihrer pharmazeutisch verwendbaren Derivate, Salze, Solvate, Tautomeren und Stereoisomeren, **dadurch gekennzeichnet, daß** man
a) eine Verbindung der Formel II 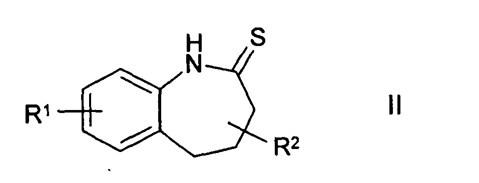 worin R¹ und R² die in Anspruch 1 angegebene Bedeutung hat,
mit einer Verbindung der Formel III 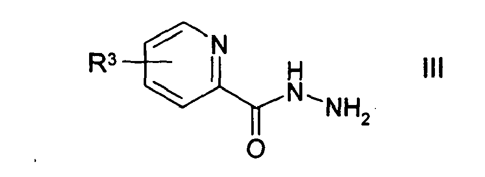 worin R³ die in Anspruch 1 angegebene Bedeutung hat,
umsetzt, oder
b) einen Rest R¹ in einen anderen Rest R¹ umwandelt, indem man einen Ether spaltet,
und/oder
eine Base oder Säure der Formel I in eines ihrer Salze umwandelt.

9. Arzneimittel, enthaltend mindestens eine Verbindung der Formel I nach einem oder mehreren der Ansprüche 1 bis 7 und/oder ihre pharmazeutisch verwendbaren Derivate, Solvate, Salze und Stereoisomere, einschließlich deren Mischungen in allen Verhältnissen, sowie gegebenenfalls Träger- und/oder Hilfsstoffe.

10. Verwendung von Verbindungen gemäß einem oder mehreren der Ansprüche 1 bis 7 sowie ihrer pharmazeutisch verwendbaren Derivate, Salze, Solvate, Tautomeren und Stereoisomeren, einschließlich deren Mischungen in allen Verhältnissen, zur Herstellung eines Arzneimittels zur Behandlung und/oder Bekämpfung von Krebs, Tumorwachstum, Metastasenwachstum, Fibrose, Restenose, HIV Infektion, Alzheimer, Atherosklerose, und/oder zur Förderung der Wundheilung.

11. Verwendung nach Anspruch 10, wobei der Tumor ausgewählt ist aus der Gruppe der Tumoren des Plattenepithel, der Blasen, des Magens, der Nieren, von Kopf und Hals, des Ösophagus, des Gebärmutterhals, der Schilddrüse, des Darms, der Leber, des Gehirns, der Prostata, des Urogenitaltrakts, des lymphatischen Systems, des Magens, des Kehlkopfs, der Lunge, Lungenadenokarzinom, kleinzelliges Lungenkarzinom, Bauchspeicheldrüsenkrebs, Glioblastom, Kolonkarzinom, Brustkarzinom, Tumor des Blut- und Immunsystems, akute myelotische Leukämie, chronische myelotische Leukämie, akute lymphatische Leukämie, chronische lymphatische Leukämie.

12. Verwendung von Verbindungen gemäß Anspruch 10 und/oder ihrer physiologisch unbedenklichen Salze und Solvate zur Herstellung eines Arzneimittels zur Behandlung von festen Tumoren, wobei eine therapeutisch wirksame Menge einer Verbindung der Formel I in Kombination mit einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

13. Verwendung von Verbindungen gemäß Anspruch 10 und/oder ihrer physiologisch unbedenklichen Salze und Solvate zur Herstellung eines Arzneimittels zur Behandlung von festen Tumoren wobei eine therapeutisch wirksame Menge einer Verbindung der Formel I in Kombination mit Radiotherapie und einer Verbindung aus der Gruppe 1) Östrogenrezeptormodulator, 2) Androgenrezeptormodulator, 3) Retinoidrezeptormodulator, 4) Zytotoxikum, 5) antiproliferatives Mittel, 6) Prenyl-Proteintransferasehemmer, 7) HMG-CoA-Reduktase-Hemmer, 8) HIV-Protease-Hemmer, 9) Reverse-Transkriptase-Hemmer sowie 10) weiterer Angiogenese-Hemmer verabreicht wird.

14. Arzneimittel enthaltend mindestens eine Verbindung der Formel I gemäß einem oder mehreren der Ansprüche 1 bis 7 und/oder ihre pharmazeutisch verwendbaren Derivate, Solvate und Stereoisomere, einschließlich deren Mischungen in allen Verhältnissen, und mindestens einen weiteren Arzneimittelwirkstoff.

## Claims

1. Compounds of the formula I 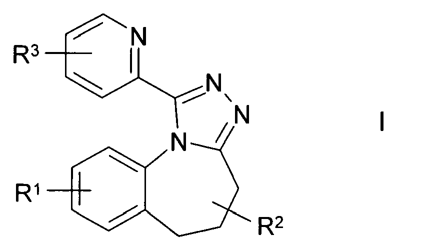 in which
R¹ denotes H, A, OH, OA, NO₂, NH₂, NHA, NA₂, Hal, CN, A-COO, COOH, COOA or CONR⁴R⁵,
R², R³ each, independently of one another, denote H, A, alkenyl having 2-6 C atoms, alkynyl having 2-6 C atoms or Hal,
R⁴, R⁵ each, independently of one another, denote H or A,
A denotes unbranched or branched alkyl having 1, 2, 3, 4, 5, 6, 7, 8, 9 or 10 C atoms, in which 1-7 H atoms may be replaced by F,
Hal denotes F, Cl, Br or I,
and pharmaceutically usable derivatives, solvates, salts, tautomers and stereoisomers thereof, including mixtures thereof in all ratios.

2. Compounds according to Claim 1 in which
R¹ denotes H, OH, OA, Hal, CN or A-COO,
and pharmaceutically usable derivatives, solvates, salts and stereoisomers thereof, including mixtures thereof in all ratios.

3. Compounds according to Claim 1 or 2 in which
R² denotes H or A,
and pharmaceutically usable derivatives, solvates, salts and stereoisomers thereof, including mixtures thereof in all ratios.

4. Compounds according to Claim 1, 2 or 3 in which
R³ denotes A,
and pharmaceutically usable derivatives, solvates, salts and stereoisomers thereof, including mixtures thereof in all ratios.

5. Compounds according to one or more of Claims 1-4 in which
A denotes unbranched or branched alkyl having 1, 2, 3, 4, 5 or 6 C atoms, in which 1-5 H atoms may be replaced by F,
and pharmaceutically usable derivatives, solvates, salts and stereoisomers thereof, including mixtures thereof in all ratios.

6. Compounds according to one or more of Claims 1-5 in which
R¹ denotes H, OH, OA, Hal, CN or A-COO,
R² denotes H or A,
R³ denotes A,
A denotes unbranched or branched alkyl having 1, 2, 3, 4, 5 or 6 C atoms, in which 1-5 H atoms may be replaced by F,
Hal denotes F, Cl, Br or I,
and pharmaceutically usable derivatives, solvates, salts and stereoisomers thereof, including mixtures thereof in all ratios.

7. Compounds according to Claim 1 selected from the group
| No. | Name and/or structure |
|---|---|
| "A1" | 8-Methoxy-4-methyl-1-(6-methylpyridin-2-yl)-5,6-dihydro-4*H*-2,3,10b-triazabenzo[*e*]azulene |
| "A2" | 8-Hydroxy-4-methyl-1-(6-methylpyridin-2-yl)-5,6-dihydro-4*H*-2,3,10b-triazabenzo[*e*]azulene |
| "A3" | 8-Fluoro-5-methyl-1-(6-methylpyridin-2-yl)-5,6-dihydro-4*H*-2,3,10b-triazabenzo[e]azulene |
| "A4" | 8-Chloro-6-methyl-1-(6-methylpyridin-2-yl)-5,6-dihydro-4*H*-2,3,10b-triazabenzo[e]azulene |
| "A5" | 8-Hydroxy-6-methyl-1-(6-methylpyridin-2-yl)-5,6-dihydro-4*H*-2,3,10b-triazabenzo[e]azulene, trifluoroacetate |
| | 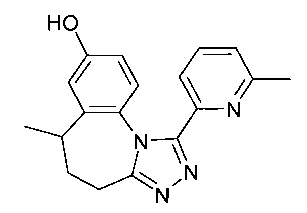 |
| "A6" | 1-(6-Methylpyridin-2-yl)-5,6-dihydro-4*H*-2,3,10b-triaza-benzo[e]azulene, trifluoroacetate |
| | 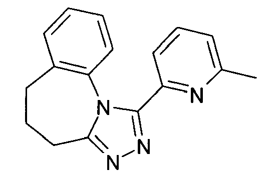 |
| "A7" | 8-Bromo-4-methyl-1-(6-methylpyridin-2-yl)-5,6-dihydro-4*H*-2,3,10b-triazabenzo[*e*]azulene |
| | 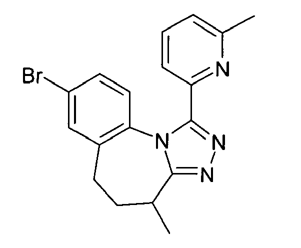 |
| "A8" | 4-Methyl-1-(6-methylpyridin-2-yl)-5,6-dihydro-4*H*-2,3,10b-tri-azabenzo[e]azulene, bistrifluoroacetate |
| "A9" | 6-Methyl-1-(6-methylpyridin-2-yl)-5,6-dihydro-4*H*-2,3,10b-tri-azabenzo[e]azulene |
| "A10" | 8-Bromo-1-(6-methylpyridin-2-yl)-5,6-dihydro-4*H*-2,3,10b-tri-azabenzo[e]azulene, trifluoroacetate |
| "A11" | 8-Bromo-5-methyl-1-(6-methylpyridin-2-yl)-5,6-dihydro-4*H*-2,3,10b-triazabenzo[e]azulene |
| "A12" | 7-Methoxy-1-(6-methylpyridin-2-yl)-5,6-dihydro-4*H*-2,3,10b-triazabenzo[e]azulene |
| "A13" | 7-Hydroxy-1-(6-methylpyridin-2-yl)-5,6-dihydro-4*H*-2,3,10b-triazabenzo[e]azulene |
| "A14" | 8-Cyano-4-methyl-1-(6-methylpyridin-2-yl)-5,6-dihydro-4*H*-2,3,10b-triazabenzo[e]azulene, trifluoroacetate |
| "A15" | 8-Cyano-5-methyl-1-(6-methylpyridin-2-yl)-5,6-dihydro-4*H*-2,3,10b-triazabenzo[e]azulene, trifluoroacetate |
| "A16" | 7-Acetoxy-1-(6-methylpyridin-2-yl)-5,6-dihydro-4*H*-2,3,10b-triazabenzo[e]azulene |
| "A17" | 8-Chloro-5-methyl-1-(6-methylpyridin-2-yl)-5,6-dihydro-4*H*-2,3,10b-triazabenzo[e]azulene |
| "A18" | 8-Methoxy-6-methyl-1-(6-methylpyridin-2-yl)-5,6-dihydro-4*H*-2,3,10b-thazabenzo[e]azulene, trifluoroacetate |
| "A19" | 8-Hydroxy-4-ethyl-1-(6-methylpyridin-2-yl)-5,6-dihydro-4*H*-2,3,10b-triazabenzo[e]azulene |
| "A20" | 8-Hydroxy-4-propyl-1-(6-methylpyridin-2-yl)-5,6-dihydro-4*H*-2,3,10b-triazabenzo[e]azulene |
| "A21" | 8-Hydroxy-4-isobutyl-1-(6-methylpyridin-2-yl)-5,6-dihydro-4*H*-2,3,10b-triazabenzo[e]azulene |
| | |
and pharmaceutically usable derivatives, solvates, salts and stereoisomers thereof, including mixtures thereof in all ratios.

8. Process for the preparation of compounds of the formula I according to Claims 1-7 and pharmaceutically usable derivatives, salts, solvates, tautomers and stereoisomers thereof, **characterised in that**
a) a compound of the formula II 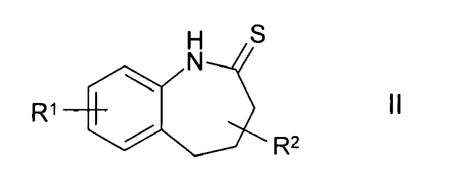 in which R¹ and R² have the meaning indicated in Claim 1,
is reacted with a compound of the formula III 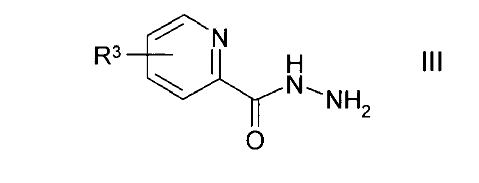 in which R³ has the meaning indicated in Claim 1,
or
b) a radical R¹ is converted into another radical R¹ by cleaving an ether,
and/or
a base or acid of the formula I is converted into one of its salts.

9. Medicament comprising at least one compound of the formula I according to one or more of Claims 1 to 7 and/or pharmaceutically usable derivatives, solvates, salts and stereoisomers thereof, including mixtures thereof in all ratios, and optionally excipients and/or adjuvants.

10. Use of compounds according to one or more of Claims 1 to 7 and pharmaceutically usable derivatives, salts, solvates, tautomers and stereoisomers thereof, including mixtures thereof in all ratios, for the preparation of a medicament for the treatment and/or combating of cancer, tumour growth, metastatic growth, fibrosis, restenosis, HIV infection, Alzheimer's, atherosclerosis, and/or for promoting wound healing.

11. Use according to Claim 10, where the tumour is selected from the group of tumours of the squamous epithelium, the bladder, the stomach, the kidneys, of head and neck, the oesophagus, the cervix, the thyroid, the intestine, the liver, the brain, the prostate, the urogenital tract, the lymphatic system, the stomach, the larynx, the lung, lung adenocarcinoma, small-cell lung carcinoma, pancreatic cancer, glioblastoma, colon carcinoma, breast carcinoma, tumour of the blood and immune system, acute myeloid leukaemia, chronic myeloid leukaemia, acute lymphatic leukaemia, chronic lymphatic leukaemia.

12. Use of compounds according to Claim 10 and/or physiologically acceptable salts and solvates thereof for the preparation of a medicament for the treatment of solid tumours, where a therapeutically effective amount of a compound of the formula I is administered in combination with a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitor.

13. Use of compounds according to Claim 10 and/or physiologically acceptable salts and solvates thereof for the preparation of a medicament for the treatment of solid tumours, where a therapeutically effective amount of a compound of the formula I is administered in combination with radiotherapy and a compound from the group 1) oestrogen receptor modulator, 2) androgen receptor modulator, 3) retinoid receptor modulator, 4) cytotoxic agent, 5) antiproliferative agent, 6) prenyl-protein transferase inhibitor, 7) HMG-CoA reductase inhibitor, 8) HIV protease inhibitor, 9) reverse transcriptase inhibitor and 10) further angiogenesis inhibitor.

14. Medicament comprising at least one compound of the formula I according to one or more of Claims 1 to 7 and/or pharmaceutically usable derivatives, solvates and stereoisomers thereof, including mixtures thereof in all ratios, and at least one further medicament active compound.

## Revendications

1. Composés de formule I 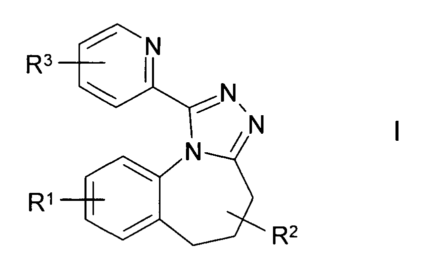 dans laquelle
R¹ désigne H, A, OH, OA, NO₂, NH₂, NHA, NA₂, Hal, CN, A-COO, COOH, COOA ou CONR⁴R⁵,
R², R³ désignent chacun, indépendamment l'un de l'autre, H, A, alcényle ayant 2-6 atomes de C, alcynyle ayant 2-6 atomes de C ou Hal,
R⁴, R⁵ désignent chacun, indépendamment l'un de l'autre, H ou A,
A désigne alkyle non ramifié ou ramifié ayant 1, 2, 3, 4, 5, 6, 7, 8, 9 ou 10 atomes de C, où 1-7 atomes de H peu- vent être remplacés par F,
Hal désigne F, Cl, Br ou I,
et les dérivés, solvats, sels, tautomères et stéréoisomères pharmaceutiquement utilisables de ceux-ci, y compris des mélanges de ceux-ci en toutes proportions.

2. Composés selon la revendication 1, dans lesquels
R¹ désigne H, OH, OA, Hal, CN ou A-COO,
et les dérivés, solvats, sels et stéréoisomères pharmaceutiquement utilisables de ceux-ci, y compris des mélanges de ceux-ci en toutes proportions.

3. Composés selon la revendication 1 ou 2, dans lesquels
R² désigne H ou A,
et les dérivés, solvats, sels et stéréoisomères pharmaceutiquement utilisables de ceux-ci, y compris des mélanges de ceux-ci en toutes proportions.

4. Composés selon la revendication 1, 2 ou 3, dans lesquels
R³ désigne A,
et les dérivés, solvats, sels et stéréoisomères pharmaceutiquement utilisables de ceux-ci, y compris des mélanges de ceux-ci en toutes proportions.

5. Composés selon l'une ou plusieurs des revendications 1-4, dans lesquels
A désigne alkyle non ramifié ou ramifié ayant 1, 2, 3, 4, 5 ou 6 atomes de C, où 1-5 atomes de H peuvent être remplacés par F,
et les dérivés, solvats, sels et stéréoisomères pharmaceutiquement utilisables de ceux-ci, y compris des mélanges de ceux-ci en toutes proportions.

6. Composés selon l'une ou plusieurs des revendications 1-5, dans lesquels
R¹ désigne H, OH, OA, Hal, CN ou A-COO,
R² désigne H ou A,
R³ désigne A,
A désigne alkyle non ramifié ou ramifié ayant 1, 2, 3, 4, 5 ou 6 atomes de C, où 1-5 atomes de H peuvent être remplacés par F,
Hal désigne F, Cl, Br ou I,
et les dérivés, solvats, sels et stéréoisomères pharmaceutiquement utilisables de ceux-ci, y compris des mélanges de ceux-ci en toutes proportions.

7. Composés selon la revendication 1, choisis dans le groupe constitué par
| n° | Nom et/ou structure |
|---|---|
| "A1" | 8-Méthoxy-4-méthyl-1-(6-méthylpyridin-2-yl)-5,6-dihydro-4*H*-2,3,10b-triazabenzo[*e*]azulène |
| "A2" | 8-Hydroxy-4-méthyl-1-(6-méthylpyridin-2-yl)-5,6-dihydro-4*H*-2,3,10b-triazabenzo[*e*]azulène |
| "A3" | 8-Fluoro-5-méthyl-1-(6-méthylpyridin-2-yl)-5,6-dihydro-4*H*-2,3,10b-triazabenzo[e]azulène |
| "A4" | 8-Chloro-6-méthyl-1-(6-méthylpyridin-2-yl)-5,6-dihydro-4*H*-2,3,10b-triazabenzo[e]azulène |
| "A5" | 8-Hydroxy-6-méthyl-1-(6-méthylpyridin-2-yl)-5,6-dihydro-4*H*-2,3,10b-triazabenzo[e]azulène, trifluoroacétate |
| | 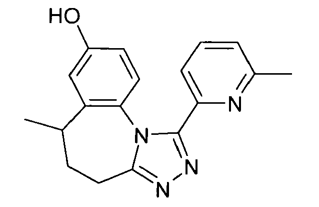 |
| "A6" | 1-(6-Méthylpyridin-2-yl)-5,6-dihydro-4*H*-2,3,10b-triaza-benzo[e]azulène, trifluoroacétate |
| | 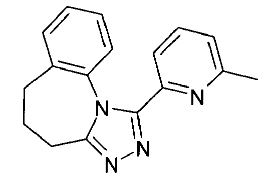 |
| "A7" | 8-Bromo-4-méthyl-1-(6-méthylpyridin-2-yl)-5,6-dihydro-4*H*-2,3,10b-triazabenzo[e]azulène |
| | 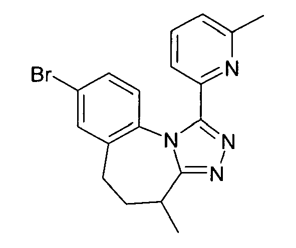 |
| "A8" | 4-Méthyl-1-(6-méthylpyridin-2-yl)-5,6-dihydro-4*H*-2,3,10b-triazabenzo[e]azulène, bistrifluoroacétate |
| "A9" | 6-Méthyl-1-(6-méthylpyridin-2-yl)-5,6-dihydro-4*H*-2,3,10b-triazabenzo[e]azulène |
| "A10" | 8-Bromo-1-(6-méthylpyridin-2-yl)-5,6-dihydro-4*H*-2,3,10b-triazabenzo[e]azulène, trifluoroacétate |
| "A11" | 8-Bromo-5-méthyl-1-(6-méthylpyridin-2-yl)-5,6-dihydro-4*H*-2,3,10b-triazabenzo[e]azulène |
| "A12" | 7-Méthoxy-1-(6-méthylpyridin-2-yl)-5,6-dihydro-4*H*-2,3,10b-triazabenzo[e]azulène |
| "A13" | 7-Hydroxy-1-(6-méthylpyridin-2-yl)-5,6-dihydro-4*H*-2,3,10b-triazabenzo[e]azulène |
| "A14" | 8-Cyano-4-méthyl-1-(6-méthylpyridin-2-yl)-5,6-dihydro-4*H*-2,3,10b-triazabenzo[e]azulène, trifluoroacétate |
| "A15" | 8-Cyano-5-méthyl-1-(6-méthylpyridin-2-yl)-5,6-dihydro-4*H*-2,3,10b-triazabenzo[e]azulène, trifluoroacétate |
| "A16" | 7-Acétoxy-1-(6-méthylpyridin-2-yl)-5,6-dihydro-4*H*-2,3,10b-triazabenzo[e]azulène |
| "A17" | 8-Chloro-5-méthyl-1-(6-méthylpyridin-2-yl)-5,6-dihydro-4*H*-2,3,10b-triazabenzo[e]azulène |
| "A18" | 8-Méthoxy-6-méthyl-1-(6-méthylpyridin-2-yl)-5,6-dihydro-4*H*-2,3,10b-triazabenzo[e]azulène, trifluoroacétate |
| "A19" | 8-Hydroxy-4-éthyl-1-(6-méthylpyridin-2-yl)-5,6-dihydro-4*H*-2,3,10b-triazabenzo[e]azulène |
| "A20" | 8-Hydroxy-4-propyl-1-(6-méthylpyridin-2-yl)-5,6-dihydro-4*H*-2,3,10b-triazabenzo[e]azulène |
| "A21" | 8-Hydroxy-4-isobutyl-1-(6-méthylpyridin-2-yl)-5,6-dihydro-4*H*-2,3,10b-triazabenzo[e]azulène |
| | |
et les dérivés, solvats, sels et stéréoisomères pharmaceutiquement utilisables de ceux-ci, y compris des mélanges de ceux-ci en toutes proportions.

8. Procédé de préparation de composés de formule I selon les revendications 1-7 et de dérivés, sels, solvats, tautomères et stéréoisomères pharmaceutiquement utilisables de ceux-ci, **caractérisé en ce que**
a) un composé de formule II 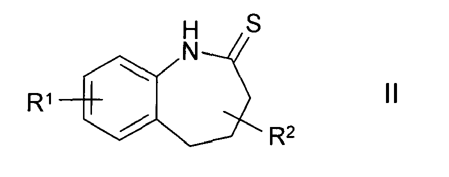 dans laquelle R¹ et R² revêtent la signification indiquée selon la revendication 1,
est réagi avec un composé de formule III 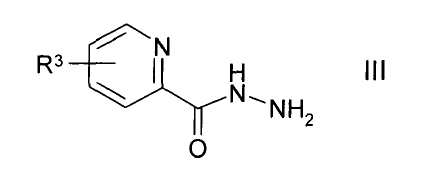 dans laquelle R³ revêt la signification indiquée selon la revendication 1,
ou
b) un radical R¹ est converti en un autre radical R¹ par clivage d'un éther,
et/ou
une base ou un acide de formule I est converti(e) en l'un de ses sels.

9. Médicament comprenant au moins un composé de formule I selon l'une ou plusieurs des revendications 1 à 7 et/ou des dérivés, solvats, sels et stéréoisomères pharmaceutiquement utilisables de celui-ci, y compris des mélanges de ceux-ci en toutes proportions, et éventuellement des excipients et/ou des adjuvants.

10. Utilisation de composés selon l'une ou plusieurs des revendications 1 à 7 et de dérivés, sels, solvats, tautomères et stéréoisomères pharmaceutiquement utilisables de ceux-ci, y compris des mélanges de ceux-ci en toutes proportions,
pour la préparation d'un médicament destiné au traitement et/ou à la lutte contre le cancer, la croissance tumorale, la croissance métastatique, la fibrose, la resténose, l'infection par le VIH, la maladie d'Alzheimer, l'athérosclérose, et/ou destiné à favoriser la guérison de plaies.

11. Utilisation selon la revendication 10, dans laquelle la tumeur est choisie dans le groupe constitué par les tumeurs de l'épithélium squameux, de la vessie, de l'estomac, des reins, de la tête et du cou, de l'oesophage, du col de l'utérus, de la thyroïde, des intestins, du foie, du cerveau, de la prostate, du tractus urogénital, du système lymphatique, de l'estomac, du larynx, du poumon, l'adénocarcinome du poumon, les carcinomes du poumon à petites cellules, le cancer du pancréas, les glioblastomes, le carcinome du côlon, le carcinome du sein, une tumeur du sang et du système immunitaire, la leucémie myéloïde aiguë, la leucémie myéloïde chronique, la leucémie lymphatique aiguë, la leucémie lymphatique chronique.

12. Utilisation de composés selon la revendication 10 et/ou de sels et solvats physiologiquement acceptables de ceux-ci pour la préparation d'un médicament destiné au traitement de tumeurs solides, dans laquelle une quantité thérapeutiquement efficace d'un composé de formule I est administrée en association avec un composé issu du groupe 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) un autre inhibiteur de l'angiogenèse.

13. Utilisation de composés selon la revendication 10 et/ou de sels et solvats physiologiquement acceptables de ceux-ci pour la préparation d'un médicament destiné au traitement de tumeurs solides, dans laquelle une quantité thérapeutiquement efficace d'un composé de formule I est administrée en association avec une radiothérapie et un composé issu du groupe 1) un modulateur du récepteur des oestrogènes, 2) un modulateur du récepteur des androgènes, 3) un modulateur du récepteur des rétinoïdes, 4) un agent cytotoxique, 5) un agent antiprolifératif, 6) un inhibiteur de la prényl-protéine transférase, 7) un inhibiteur de la HMG-CoA réductase, 8) un inhibiteur de la protéase du VIH, 9) un inhibiteur de la transcriptase inverse et 10) un autre inhibiteur de l'angiogenèse.

14. Médicament comprenant au moins un composé de formule I selon l'une ou plusieurs des revendications 1 à 7 et/ou des dérivés, solvats et stéréoisomères pharmaceutiquement utilisables de celui-ci, y compris des mélanges de ceux-ci en toutes proportions, et au moins un autre composé actif médicamenteux.
